(19) Europäisches Patentamt
European Patent Office
Office européen des brevets

(11) **EP 1 093 798 B1**

(12) **FASCICULE DE BREVET EUROPEEN**

(45) Date de publication et mention de la délivrance du brevet:
**31.08.2005 Bulletin 2005/35**

(51) Int Cl.[7]: **A61K 7/42**, A61K 7/40, A61K 7/02, A61K 7/06

(21) Numéro de dépôt: **00402810.6**

(22) Date de dépôt: **11.10.2000**

(54) **Emulsions sans émulsionnant contenant au moins un filtre uv organique insoluble**

Mindestens ein unlösliches organisches UV-Filter enthaltende Emulsionen ohne Emulgatoren

Emulsions without emulsifiers containing at least one insoluble organic UV-filter

(84) Etats contractants désignés:
**AT BE CH CY DE DK ES FI FR GB GR IE IT LI LU MC NL PT SE**

(30) Priorité: **21.10.1999 FR 9913221**

(43) Date de publication de la demande:
**25.04.2001 Bulletin 2001/17**

(73) Titulaire: **L'OREAL**
**75008 Paris (FR)**

(72) Inventeurs:
- **Candau, Didier**
**91570 Biebres (FR)**
- **Pisson, Anne-Marie**
**91800 Boussy Saint Antoine (FR)**

(74) Mandataire: **Miszputen, Laurent**
**L'OREAL - D.I.P.I.**
**25-29 Quai Aulagnier**
**92600 Asnières (FR)**

(56) Documents cités:
**EP-A- 0 949 251** **EP-A- 0 987 006**
**WO-A-97/03643**

- **DATABASE CHEMICAL ABSTRACTS [en ligne] STN; abrégé: 122: 64 010, XP002143606 & JP 06 279252 A (NOEVIR KK) 4 octobre 1994 (1994-10-04)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 093 798 B1

## Description

La présente invention a pour objet une émulsion cosmétique ou dermatologique sans émulsionnant, caractérisée par le fait qu'elle comporte :

(a) au moins une phase aqueuse et
(b) au moins une phase grasse ;
(c) au moins un système photoprotecteur capable de filtrer les rayons UV contenant au moins un filtre UV organique insoluble dans ladite émulsion.

L'invention concerne également ses utilisations dans et pour la fabrication de compositions cosmétiques ou dermatologiques pour la photoprotection de la peau ou des cheveux.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination d'UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions antisolaires se présentent assez souvent sous la forme d'une émulsion, de type huile-dans-eau (c'est à dire un support cosmétiquement et/ou dermatologiquement acceptable constitué d'une phase continue dispersante aqueuse et d'une phase discontinue dispersée grasse) ou eau-dans-huile (phase aqueuse dispersée dans une phase grasse continue), qui contient, à des concentrations diverses, un ou plusieurs filtres organiques classiques, lipophiles et/ou et/ou des nanopigments minéraux d'oxydes metalliques, capables d'absorber sélectivement les rayonnements UV nocifs, ces filtres (et leurs quantités) étant sélectionnés en fonction du facteur de protection solaire recherché (le facteur de protection solaire (SPF) s'exprimant mathématiquement par le rapport du temps d'irradiation nécessaire pour atteindre le seuil érythématogène avec le filtre UV au temps nécessaire pour atteindre le seuil érythématogène sans filtre UV). Dans de telles émulsions, les filtres hydrophiles sont présents dans la phase aqueuse et les filtres lipophiles sont présents dans la phase grasse.

Les émulsions huile-dans-eau sont, d'une manière générale, plus appréciées par le consommateur que les émulsions eau-dans-huile, en raison notamment de leur toucher agréable (voisin de eau) et de leur présentation sous forme de lait ou de crème non gras; cependant, elles perdent également plus facilement leur efficacité en protection UV dès lors qu'elles viennent en contact avec l'eau ; en effet, les filtres hydrophiles, ont tendance à disparaître à l'eau, par baignade en mer ou en piscine, sous la douche ou lors de la pratique de sports nautiques ; ainsi, les compositions solaires qui les contiennent, seuls ou associés aux filtres lipophiles, n'apportent plus la protection initiale recherchée dès lors que le substrat (peau ou cheveu) sur lequel elles ont été appliquées vient en contact avec l'eau.

On peut disposer de compositions antisolaires présentant une résistance à l'eau améliorée en mettant en oeuvre des émulsions eau-dans-huile. En effet, un filtre hydrophile est plus rémanent à l'eau au sein d'une émulsion eau-dans-huile qu'au sein d'une émulsion huile-dans-eau. Cependant, comme il a été indiqué plus haut, de telles compositions ne donnent pas encore entièrement satisfaction dans la mesure où elles laissent après application une impression de gras particulièrement désagréable pour l'utilisateur.

Ainsi, le besoin subsiste toujours quant à pouvoir disposer de compositions antisolaires apportant à ia peau et/ou aux cheveux une protection solaire efficace, stable dans le temps et résistante à l'eau (rémanence à l'eau) et dont les performances cosmétiques seraient comparables à celles obtenues avec les émulsions huile/eau classiques.

La Demanderesse a découvert de manière surprenante et inattendue que des émulsions sans émulsionnant contenant au moins un filtre UV organique insoluble dans lesdites émulsions permettaient non seulement d'obtenir des compositions antisolaires dont les performances cosmétiques étaient comparables à celles obtenues généralement avec une composition antisolaire classique sous forme d'émulsion huile/eau mais aussi présentaient une bonne stabilité ainsi qu'une rémanence à l'eau améliorée.

Ces découvertes sont à l'origine de la présente invention.
La présente invention a pour objet une émulsion cosmétique ou dermatologique sans émulsionnant, caractérisée par le fait qu'elle comporte :

(a) au moins une phase aqueuse et
(b) au moins une phase grasse ;
(c) au moins un système photoprotecteur capable de filtrer les rayons UV contenant au moins un filtre UV organique insoluble dans ladite émulsion , sous forme micronisée de taille de particule moyenne allant de 0,01 à 2 µm.

Par émulsion cosmétique ou dermatologique, au sens de la présente invention et dans le texte qui suit, on entend toute émulsion dont la phase aqueuse et la phase grasse sont constituées de substances cosmétiquement ou dermatologiquement acceptables pour une application topique sur les matières kératiniques humaines incluant la peau, les cheveux, les cils, les sourcils, les lèvres, les ongles ou les muqueuses.
Par émulsion sans émulsionnant, au sens de la présente invention et dans le texte qui suit, on entend toute émulsion huile-dans-eau (constituée d'une phase continue dispersante aqueuse et d'une phase discontinue dispersée grasse) ou toute émulsion eau-dans-huile (constituée d'une phase continue dispersante grasse et d'une phase discontinue dispersée aqueuse) ne contenant pas de tensio-actif classique émulsionnant.
Par filtre UV organique insoluble, on entend, au sens de la présente invention, des filtres UV organiques insolubles dans les milieux cosmétiques généralement utilisés dans les formulations solaires et plus particulièrement dont la solubilité dans l'eau à 25°C est inférieure à 0,1 % en poids et dont la solubilité dans l'huile de paraffine à 25°C est inférieure à 1% en poids.
Par système photoprotecteur capable de filtrer les rayons UV, on entend par tout système constitué d'un ou plusieurs composés organiques et/ou composés minéraux filtrant les radiations UVA et/ou UV-B.
La présente invention a également pour objet l'utilisation d'une émulsion sans émulsionnant telle que définie ci-dessus pour la fabrication de compositions cosmétiques pour la protection de la peau et/ou des cheveux contre le rayonnement ultraviolet, en particulier le rayonnement solaire.
D'autres caractéristiques, aspects et avantages de la présente invention apparaîtront à la lecture de la description détaillée qui va suivre.
Les filtres UV organiques insolubles selon l'invention se présentent sous forme insoluble micronisée et la taille moyenne des particules varie de 0,01 à 2µm et plus préférentiellement de 0,02 à 1,5 µm et plus particulièrement de 0,03 à 1,0 µm.
Ils peuvent être amenés sous la forme particulaire souhaitée par tout moyen ad-hoc tel que notamment broyage à sec ou en milieu solvant, tamisage, atomisation, micronisation, pulvérisation.
Les filtres organiques insolubles selon l'invention sous forme micronisée, peuvent notamment être obtenus par un procédé de broyage d'un filtre UV insoluble sous forme de particules de taille grossière en présence d'un tensio-actif approprié permettant d'améliorer la dispersion des particules ainsi obtenues dans les formulations cosmétiques.
Un exemple de procédé de micronisation de filtres organiques insolubles est décrit dans les demandes GB-A-2 303 549, **WO97/03643** et EP-A-893 119. L'appareil de broyage utilisé selon ces documents peut être un broyeur à jet, à billes, à vibration ou à marteau et de préférence un broyeur à haute vitesse d'agitation ou un broyeur à impact et plus particulièrement un broyeur à billes rotatives, un broyeur vibrant, à broyeur à tube ou un broyeur à tige.
Selon ce procédé particulier, on utilise à titre de tensio-actifs pour le broyage desdits filtres, les alkylpolyglucosides de structure $C_nH_{2n+1}\ O(C_6H_{10}O_5)_xH$ dans laquelle n est un entier de 8 à 16 et x est le degré moyen de polymérisation de l'unité $(C_6H_{10}O_5)$ et varie de 1,4 à 1,6. Ils peuvent être choisis parmi des esters en $C_1$-$C_{12}$ d'un composé de structure $C_nH_{2n+1}\ O(C_6H_{10}O_5)_xH$ et plus précisément un ester obtenu par réaction d'un acide carboxylique en $C_1$-$C_{12}$ tel que l'acide formique, acétique, propionique, butyrique, sulfosuccinique, citrique ou tartrique avec une ou plusieurs fonctions OH libres sur l'unité glucoside $(C_6H_{10}O_5)$. Lesdits tensio-actifs sont utilisés en général à une concentration de allant de 1 à 50% en poids et plus préférentiellement de 5 à 40% en poids par rapport au filtre insoluble dans sa forme micronisée.
Les émulsions sans émulsionnant conformes à la présente invention comprennent en général au moins un polymère réticulé d'au moins un monomère à insaturation éthylénique.
Parmi les polymères réticulés d'au moins un monomère à insaturation éthylénique, conformes à l'invention, on peut citer notamment :

(i) les homopolymères ou copolymères réticulés d'au moins un monomère à insaturation éthylénique à fonction sulfonique, sous forme libre ou partiellement ou totalement salifiée ;
(ii) les copolymères réticulés d'au moins un monomère à insaturation éthylénique à fonction acide carboxylique, sous forme libre ou partiellement ou totalement salifiée ; ou d'un dérivé ester ou amide,
(iii) les homopolymères ou copolymères réticulés d'au moins un monomère à insaturation éthylénique cationique

du type ester ou amide.

Les monomères à fonction acide sulfonique sont choisis en particulier parmi l'acide 2-acrylamido-2-méthyl-propane sulfonique ainsi que ses sels.

Les monomères à fonction acide carboxylique sont choisis en particulier parmi l'acide acrylique, l'acide méthacrylique ainsi que leurs sels. Leurs esters sont choisis en général parmi ceux d'acide (meth)acrylique et alcools en $C_1$-$C_{30}$. Leurs amides sont choisis en général parmi ceux d'acide (meth)acrylique et d'amines en $C_1$-$C_{30}$ et plus particulièrement le méthacrylamide et/ou l'acrylamide

Les monomères cationiques du type ester ou amide sont choisis de préférence parmi le (méth)acrylate d'ammonium, les dialkylaminoalkyl-(meth)acrylates, en particulier, le diméthylamino-éthylmethacrylate ; les dialkyl-aminoalkyl-(meth)acrylamides ainsi que leurs sels quaternaires ou acides ; les radicaux alkyle étant, de préférence, en $C_1$-$C_4$.

Les polymères réticulés de l'invention comprennent au moins un agent de réticulation à polyinsaturation éthyléniquequi est choisi, de préférence, parmi le divinylbenzène ; le tétraallyloxyéthane ; l'éther diallylique ; les éthers polyallyliques polyglycéryliques ; les éthers allyliques d'alcools de la série du sucre comme l'érythrytol, le pentaérythrytol, l'arabitol, le sorbitol ou le glucose ; le méthylène-bis-acrylamide, l'éthylèneglycol di-(méthyl)acrylate, le di-(meth)acrylamide, le cyano-méthylacrylate, le vinyloxyéthyl-(meth)acrylate ou leurs sels métalliques.

Parmi les homopolymères ou copolymères réticulés d'au moins un monomère à insaturation éthylénique à fonction sulfonique, neutralisés à au moins 90%, on peut citer plus particulièrement

- les homopolymères réticulés d'acide 2-acrylamido-2-méthyl-propane sulfonique, neutralisés à au moins 90% tels que ceux décrits dans la demande EP-A-0815828.
- les copolymères réticulés d'acide 2-acrylamido-2-méthyl-propane sulfonique et d'acrylamide partiellement ou totalement neutralisés (par une base telle que la soude, de la potasse ou une amine) tels que décrits dans le document EP-A-503 853 et plus particulièrement dans l'exemple 1.

Parmi les copolymères réticulés d'au moins un monomère à insaturation éthylénique à fonction acide carboxylique ou d'un dérivé ester ou amide, on peut citer plus particulièrement les polymères amphiphiles non-ioniques ou anioniques comportant au moins une chaîne grasse et au moins un motif hydrophile.

Les polymères amphiphiles non-ioniques peuvent être choisis plus particulièrement parmi :

(a) les copolymères de méthacrylates ou d'acrylates d'alkyles en $C_1$-$C_6$ et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208.

(b) les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.

Les polymères amphiphiles anioniques peuvent être choisis parmi ceux dont le motif hydrophile est constitué par un monomère anionique insaturé éthylénique, plus particulièrement par un acide carboxylique vinylique et tout particulièrement par un acide acrylique, un acide méthacrylique ou leurs mélanges, et dont le motif éther d'allyl à chaîne grasse correspond au monomère de formule (I) suivante :

$$CH_2 = C(R')\ CH_2\ O\ B_n\ R \qquad \text{(I)}$$

dans laquelle R' désigne H ou $CH_3$, B désigne le radical éthylèneoxy, n est nul ou désigne un entier allant de 1 à 100, R désigne un radical hydrocarboné choisi parmi les radicaux alkyl, arylalkyl, aryl, alkylaryl, cycloalkyl, comprenant 8 à 30 atomes de carbone, de préférence 10 à 24, et plus particulièrement encore de 12 à 18 atomes de carbone. Un motif de formule (2) plus particulièrement préféré est un motif dans lequel R' désigne H, n est égal à 10, et R désigne un radical stéaryle ($C_{18}$). Des polymères amphiphiles anioniques de ce type sont décrits et préparés, selon un procédé de polymérisation en émulsion, dans le brevet EP-0216479 B2.

Parmi ces polymères amphiphiles anioniques, on préfère particulièrement selon l'invention, les polymères formés à partir de 20 à 60% en poids d'acide acrylique et/ou d'acide méthacrylique, de 5 à 60% en poids de (méth) acrylates d'alkyle inférieurs, de 2 à 50% en poids d'éther d'allyl à chaîne grasse de formule (2), et de 0 à 1% en poids d'un agent réticulant qui est un monomère insaturé polyéthylénique copolymérisable bien connu, comme le phtalate de diallyl, le (méth)acrylate d'allyl, le divinylbenzène, le diméthacrylate de (poly)éthylèneglycol, et le méthylène-bis-acrylamide.

Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous les dénominations SALCARE SC 80 et SALCARE SC90 qui sont des émulsions aqueuses à 30% d'un terpolymère réticulé d'acide méthacrylique, d'acrylate d'éthyle et de steareth-10-allyl éther (40/50/10).

Les polymères amphiphiles anioniques peuvent être également choisis parmi ceux comportant au moins un motif hydrophile de type acide carboxylique insaturé oléfinique, et au moins un motif hydrophobe exclusivement de type ester d'alkyl ($C_{10}$-$C_{30}$) d'acide carboxylique insaturé. Ils sont choisis de préférence parmi ceux dont le motif hydrophile de type acide carboxylique insaturé oléfinique correspond au monomère de formule (II) suivante :

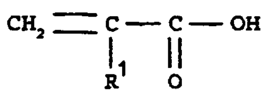

(II)

formule dans laquelle, $R^1$ désigne H ou $CH_3$ ou $C_2H_5$, c'est-à-dire des motifs acide acrylique, acide méthacrylique ou acide éthacrylique et dont le motif hydrophobe de type ester d'alkyl ($C_{10}$-$C_{30}$) d'acide carboxylique insaturé correspond au monomère de formule (III) suivante :

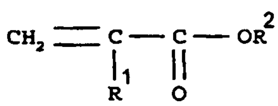

(III)

formule dans laquelle, $R^1$ désigne H ou $CH_3$ ou $C_2H_5$ (c'est-à-dire des motifs acrylates, méthacrylates ou éthacrylates) et de préférence H (motifs acrylates) ou $CH_3$ (motifs méthacrylates), $R^2$ désignant un radical alkyle en $C_{10}$-$C_{30}$, et de préférence en $C_{12}$-$C_{22}$.

Des esters d'alkyles ($C_{10}$-$C_{30}$) d'acides carboxyliques insaturés conformes à l'invention comprennent par exemple, l'acrylate de lauryle, acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.

Des polymères amphiphiles anioniques de ce type sont par exemple décrits et préparés, selon les brevets US-3 915 921 et 4 509 949.

Parmi lesdits polymères ci-dessus, on préfère tout particulièrement selon la présente invention, les produits vendus par la société GOODRICH sous les dénominations commerciales PEMULEN TR1, PEMULEN TR2, CARBOPOL 1382, et encore plus préférentiellement le PEMULEN TR1, et le produit vendu par la société S.E.P.P.I.C. sous la dénomination COATEX SX.

Parmi les homopolymères ou copolymères réticulés d'au moins un monomère à insaturation éthylénique cationique du type ester ou amide, on peut utiliser en particulier :

(1) les homopolymères d'acrylate d'ammonium ou les copolymères d'acrylate d'ammonium et d'acrylamide tels que le produit vendu sous le nom BOZEPOL C NOUVEAU ou le produit PAS 5193 vendus par la société HOECHST (ils sont décrits et préparés dans les documents FR 2 416 723, USP2798053 et USP 2 923 692) ;

(2) les homopolymères de diméthylaminoéthyl-méthacrylate quaternisé par le chlorure de méthyle tels que les produits vendus sous les noms SALCARE 95 et SALCARE 96 par la société ALLIED COLLOIDS ou les copolymères de diméthylamino-éthylméthacrylate quaternisé par le chlorure de méthyle et d'acrylamide tel que le produit SALCARE SC92 vendu par ALLIED COLLOIDS ou le produit PAS 5194 vendu par HOECHST (ils sont décrits et préparés dans le document EP-A-395.282).

Les polymères conformes à l'invention sont généralement présents dans les compositions antisolaires selon l'invention à une concentration (exprimée en matière active MA) comprise entre 0,01 et 10 % en poids, et de préférence entre 0,1 et 5 % en poids, par rapport au poids total de la composition.

Les filtres UV organiques insolubles sous forme micronisée conformes à l'invention peuvent être choisis notamment parmi les filtres UV organiques du type oxanilide, du type triazine, du type triazole, du type amide vinylique, du type cinnamide.

Parmi les filtres UV du type oxanilide conformes à l'invention, on peut citer ceux répondant à la structure :

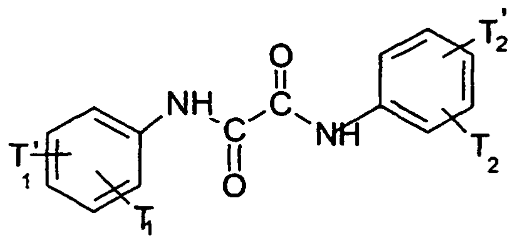

(1)

dans laquelle $T_1$ , $T'_1$, $T_2$ et $T'_2$ désignent, identiques et différents, un radical alkyle en $C_1$-$C_8$ ou un radical alcoxy en $C_1$-$C_8$ . Ces composés sont décrits dans la demande de brevet WO95/22959. A titre d'exemples , on peut citer les produits commerciaux TINUVIN 315 et TINUVIN 312 vendus par la Société CIBA-GEIGY et respectivement de structure :

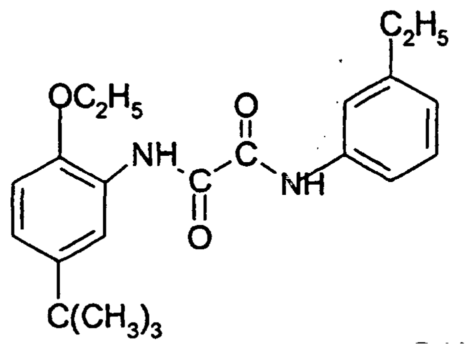

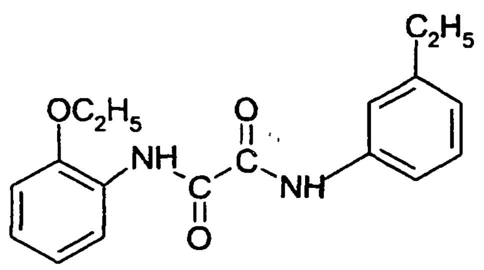

Les dérivés de 1,3,5-triazine conformes à l'invention préférentiels répondent à la formule générale suivante :

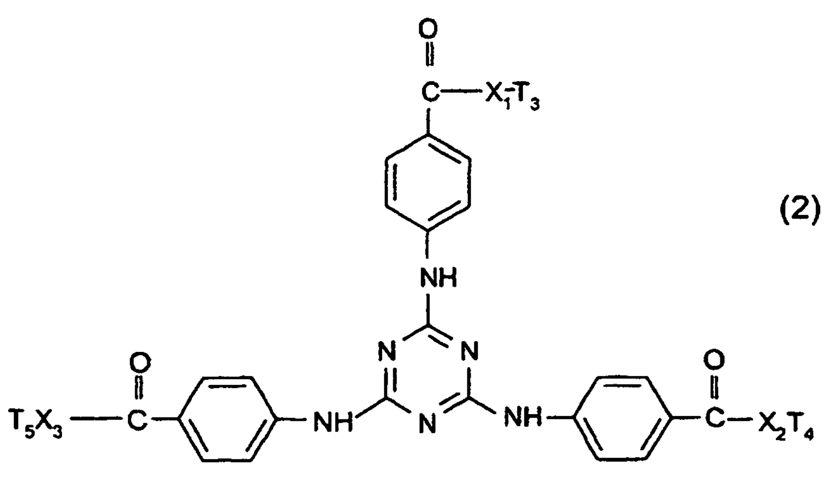

(2)

dans laquelle :

- $X_1$, $X_2$ et $X_3$, identiques ou différents, représentent l'oxygène ou un radical -NZ-;
- les radicaux Z, identiques ou différents, désignent l'hydrogène ou un radical alkyle en $C_1$-$C_{18}$, linéaire ou ramifié, un radical cycloalkyle en $C_5$-$C_{12}$ pouvant être substitué par un ou plusieurs radicaux alkyle en $C_1$-$C_4$ ;
- $T_3$, $T_4$ et $T_5$, identiques ou différents, sont choisis parmi : l'hydrogène; un métal alcalin; un radical ammonium éventuellement substitué par un ou plusieurs radicaux alkyle ou hydroxyalkyle ; un radical alkyle linéaire ou ramifié en $C_1$-$C_{18}$ ; un radical cycloalkyle en $C_5$-$C_{12}$ éventuellement substitué par un ou plusieurs radicaux alkyle en $C_1$-$C_4$ ; un radical polyoxyéthyléné comprenant de 1 à 6 unités d'oxyde d'éthylène et dont le groupe OH terminal est méthylé ; un radical de formule (3), (4) ou (5) suivantes :

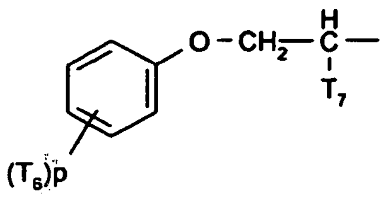

(3)

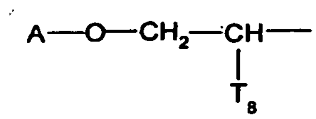

(4)

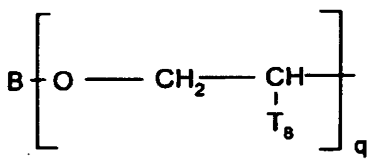

(5)

dans lesquelles :

- $T_6$ est l'hydrogène ou un radical méthyle ;
- $T_7$ est un radical alkyle en $C_1$-$C_9$;
- p est un nombre entier allant de 0 à 3 ;
- q est un nombre entier allant de 1 à 10 ;
- A est un radical alkyle en $C_4$-$C_8$ ou un radical cycloalkyle en $C_5$-$C_8$ ;
- B est choisi parmi : un radical alkyle linéaire ou ramifié en $C_1$-$C_8$ ; un radical cycloalkyle en $C_5$-$C_8$; un radical aryle éventuellement substitué par un ou plusieurs radicaux alkyle en $C_1$-$C_4$ ;
- $T_8$ est l'hydrogène ou un radical méthyle ;

Une première famille préférée de dérivés de 1,3,5-triazine est celle, notamment décrite dans le document EP-A-0517104 des 1,3,5-triazines répondant à la formule (2) ci-dessus et présentant l'ensemble des caractéristiques suivantes :

- $X_1$, $X_2$ et $X_3$ sont identiques et représentent l'oxygène ;
- $T_3$ est choisi parmi : un radical cycloalkyle en $C_5$-$C_{12}$ éventuellement substitué par un ou plusieurs radicaux alkyles en $C_1$-$C_4$ un radical de formule (2), (3) ou (4) ci-dessus dans lesquelles :
- B est un radical alkyle en $C_1$-$C_4$ ;
- $T_8$ est le radical méthyle ;
- $T_4$ et $T_5$, identiques ou différents, sont choisis parmi : l'hydrogène ; un métal alcalin ; un radical ammonium éventuellement substitué par un ou plusieurs radicaux alkyle ou hydroxyalkyle ; un radical alkyle linéaire ou ramifié en $C_1$-$C1_8$; un radical cycloalkyle en $C_5$-$C_{12}$ éventuellement substitué par un ou plusieurs radicaux alkyle en $C_1$-$C_4$ un radical de formule (3), (4) ou (5) ci-dessus dans lesquelles :

- B est un radical alkyle en $C_1$-$C_4$ ;
- $T_8$ est le radical méthyle.

Une deuxième famille préférée de dérivés de 1,3,5-triazine selon l'invention est celle, notamment décrite dans le document EP-A-0570838 des 1,3,5-triazines répondant à la formule (2) et présentant l'ensemble des caractéristiques suivantes :

- $X_1$ est l'oxygène ; $X_2$ est le radical -NH- ou l'oxygène ;
- $X_3$ est le radical -NH- ;
- $T_5$ est choisi parmi : un radical alkyle linéaire ou ramifié en $C_1$-$C_{18}$ ; un radical cycloalkyle en $C_5$-$C_{12}$ éventuellement substitué avec un ou plusieurs radicaux alkyles en $C_1$-$C_4$ ;
- $T_3$ est choisi parmi : l'hydrogène; un métal alcalin ; un radical ammonium; un radical de formule (5) ; un radical alkyle linéaire ou ramifié en $C_1$-$C_{18}$ ; un radical cycloalkyle en $C_5$-$C_{12}$ éventuellement substitué avec un ou plusieurs radicaux alkyles en $C_1$-$C_4$ ;
- si $X_2$ est le radical -NH-, alors $T_4$ est choisi parmi : un radical alkyle linéaire ou ramifié en $C_1$-$C_{18}$; un radical cycloalkyle en $C_5$-$C_{12}$ éventuellement substitué avec un ou plusieurs radicaux alkyles en $C_1$-$C_4$;
- si $X_2$ est l'oxygène, alors $T_4$ est choisi parmi l'hydrogène ; un métal alcalin ; un radical ammonium; un radical de formule (5) ; un radical alkyle linéaire ou ramifié en $C_1$-$C_{18}$ ; un radical cycloalkyle en $C_5$-$C_{12}$ éventuellement substitué avec un ou plusieurs radicaux alkyles en $C_1$-$C_4$.

Une troisième famille préférée de dérivés de 1,3,5-triazine selon l'invention est celle, notamment décrite dans le document EP-A-0796851 des 1,3,5-triazines répondant à la formule (2) et présentant l'ensemble des caractéristiques suivantes :

- $X_1$, $X_2$ et $X_3$ désignent simultanément -NZ- ;
- les radicaux Z, identiques ou différents, désignent l'hydrogène ou un radical alkyle en $C_1$-$C_{18}$, linéaire ou ramifié, un radical cycloalkyle en $C_5$-$C_{12}$ pouvant être substitué par un ou plusieurs radicaux alkyle en $C_1$-$C_4$;
- $T_3$, $T_4$ et $T_5$, identiques ou différents, désignent l'hydrogène ou un radical Z.

Ces filtres UV organiques du type triazine sont décrits dans les brevets US4617390, EP517104, EP570838.

Parmi ces filtres UV du type triazine de formule (2), on citera plus particulièrement :

- la 2,4,6-tris[p-(2'-éthylhexyl-1'-oxycarbonyl)-anilino]-1,3,5-triazine qui est un filtre connu en soi, actif dans l'UV-B, se présentant sous une forme solide, et qui est vendu notamment sous la dénomination commerciale de "UVINUL T150" par la Société BASF. Ce produit répond à la formule suivante :

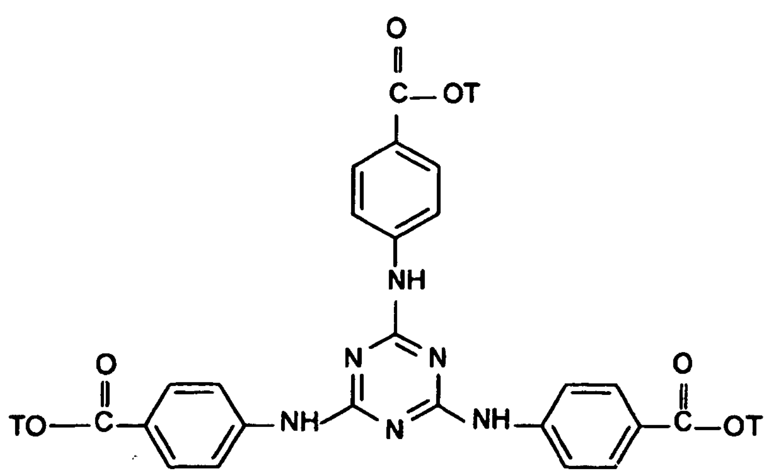

dans laquelle T désigne un radical 2-éthylhexyle ;

- la 2-[(p-(tertiobutylamido)anilino]-4,6-bis-[(p-(2'-éthylhexyl-1'-oxycarbonyl)anilino]- 1,3,5-triazine, de structure suivante :

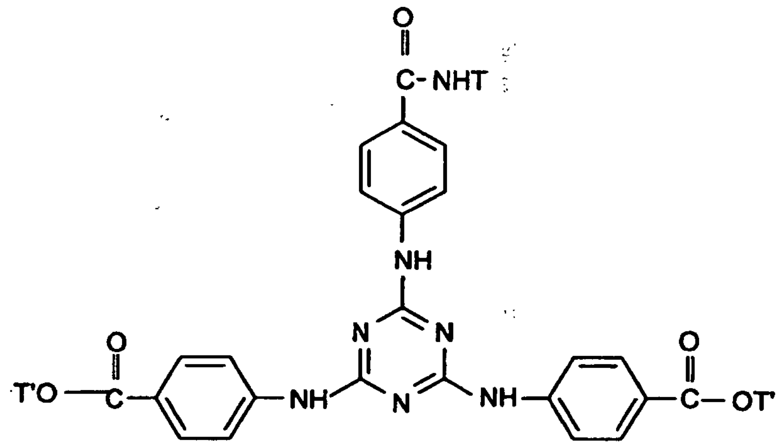

dans laquelle T' désigne un radical 2-éthylhexyle et T désigne un radical tert-butyle.

Parmi les filtres UV du type triazine conformes à l'invention, on peut également mentionner les dérivés insolubles de s-triazine portant des groupements benzalmalonates et/ou phenylcyanoacrylates tels que ceux décrits dans la demande EP-A-0790243.

Parmi ces filtres UV du type triazine, on citera plus particulièrement les composés suivants :

- la 2,4,6-tris(4'-amino benzalmalonate de diéthyle)-s-triazine,
- la 2,4,6-tris(4'-amino benzalmalonate de diisopropyle)-s-triazine,
- la 2,4,6-tris(4'-amino benzalmalonate de diméthyle)-s-triazine,
- la 2,4,6-tris($\alpha$-cyano-4-aminocinnamate d'éthyle)-s-triazine.

Parmi les filtres UV du type triazine conformes à l'invention, on peut également mentionner ceux répondant à la formule (6) suivante :

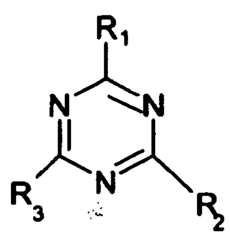

(6)

dans laquelle $R_1$, $R_2$, $R_3$,, indépendamment, sont phenyle, phenoxy, pyrrolo, dans lesquels les phenyle, phenoxy, pyrrolo sont éventuellement substitués par un, deux ou trois substituants choisis parmi OH, $C_1$-$C_{18}$alkyle ou alkoxy, $C_1$-$C_{18}$carboxyalkyle, $C_5$-$C_8$cycloalkyle, un groupe méthylidènecamphre, un groupe $-(CH=CH)_n(CO)-OR_4$ , avec $R_4$ soit $C_1$-$C_{18}$alkyle soit cinnamyle, et n vaut 0 ou 1.

Ces composés sont décrits dans WO 97/03642, GB 2286774, EP-743309, WO 98/22447, GB 2319523.

Parmi les filtres UV du type triazine conformes à l'invention, on peut encore mentionner les dérivés insolubles de s-triazine portant des groupements benzotriazoles et/ou benzothiazoles tels que ceux décrits dans la demande WO98/25922 (faisant partie intégrante du contenu de la description).

Parmi ces composés, on peut citer plus particulièrement :

- la 2,4,6-tris[(3'-benzotriazol-2-yl-2'-hydroxy-5'-methyl) phenylamino]-s-triazine,
- 2,4,6-tris[(3'-benzotriazol-2-yl-2'-hydroxy-5'-ter-octyl) phénylamino]-s-triazine.

Parmi les filtres UV organiques du type triazole conformes à l'invention, on peut citer ceux de formule suivante (7) tels que décrits dans la demande WO95/22959 :

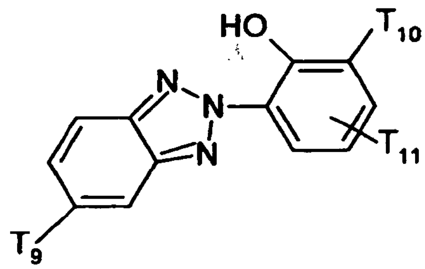

(7)

dans laquelle $T_g$ désigne un atome d'hydrogène ou un radical alkyle en $C_1$-$C_{18}$ ; $T_{10}$ et $T_{11}$, identiques ou différents, désignent un radical alkyle en $C_1$-$C_{18}$ éventuellement substitué par un phényle.

A titre d'exemple de composés de formule (7), on peut citer les produits commerciaux TINUVIN 328, 320, 234 et 350 de la Société CIBA-GEIGY de structure suivante :

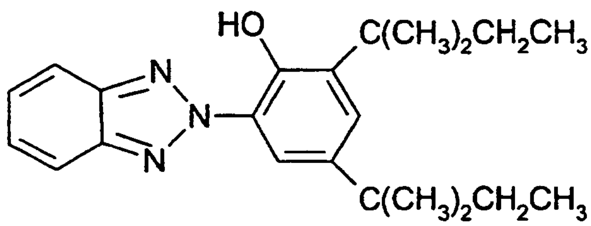

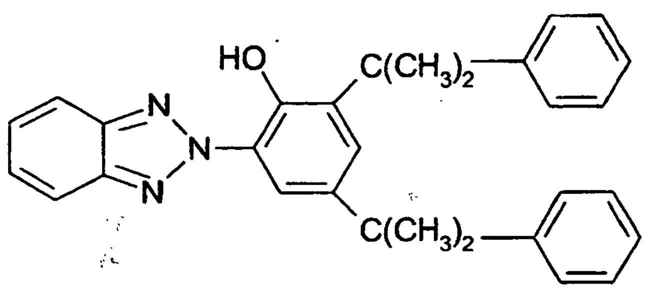

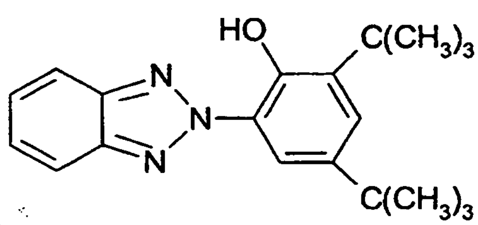

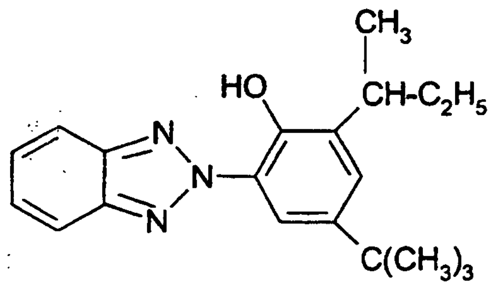

Parmi les filtres UV organiques du type triazole conformes à l'invention, on peut citer les composés tels que décrits dans les brevets US 5 687 521, US 5 687 521, US 5 373 037, US 5 362 881 et en particulier le [2 ,4'-dihydroxy-3-(2H-benzotriazol-2-yl)-5-(1,1,3,3-tétraméthylbutyl)-2'-n-octoxy-5'-benzoyl] diphénylméthane vendu sous le nom MIXXIM PB30 par la société FAIRMOUNT CHEMICAL de structure :

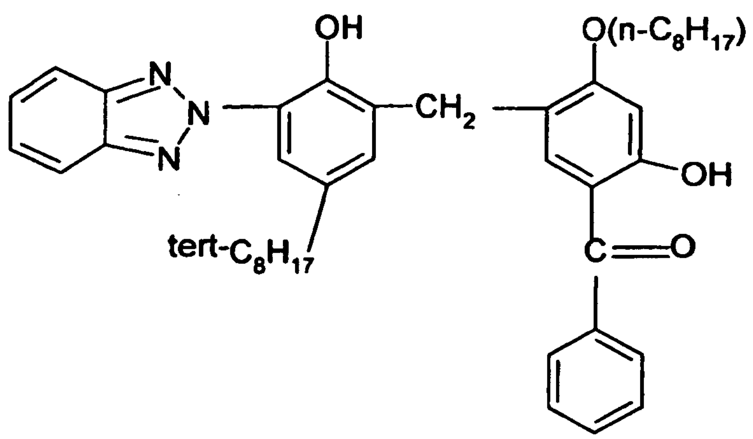

Parmi les filtres UV organiques du type benzotriazole conformes à l'invention, on peut citer les dérivés de méthylène bis-(hydroxyphényl benzotriazole) de structure suivante :

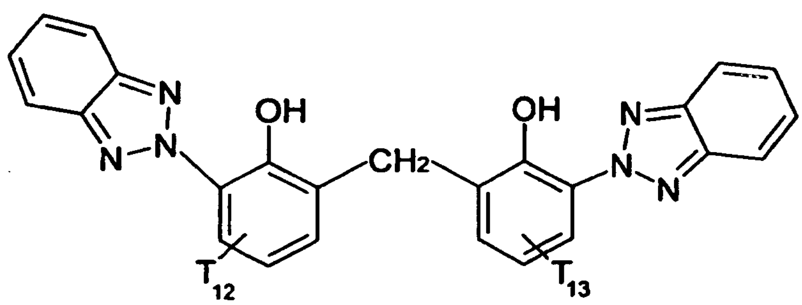

(8)

dans laquelle les radicaux $T_{12}$ et $T_{13}$, identiques ou différents, désignent un radical alkyle en $C_1$-$C_{18}$ pouvant être substitué par un ou plusieurs radicaux choisis parmi alkyle en $C_1$-$C_4$, cycloalkyle en $C_5$-$C_{12}$ ou un reste aryle. Ces composés sont connus en soi et décrits dans les demandes US 5237 071, US 5 166 355, GB-A-2 303 549, DE 197 26 184 et EP-A-893 119.

Dans la formule (8) définie ci-dessus : les groupes alkyle en $C_1$-$C_{18}$ peuvent être linéaires ou ramifiés et sont par exemple méthyle, éthyle, n-propyle, isopropyle, n-butyle, isobutyle, tert-butyle, tert-octyle, n-amyle, n-hexyle, n-heptyle, n-octyle, iso-octyle, n-nonyle, n-décyle, n-undécyle, n-dodécyle, tétradécyle, hexydécyle, ou octadécyle; les

groupes cycloalkyle en $C_5$-$C_{12}$ sont par exemple cyclopentyle, cyclohexyle, cyclooctyle ; les groupes aryle sont par exemple phényle, benzyle.

Parmi les composés de formule (8), on préfère plus particulièrement ceux de structure suivante :

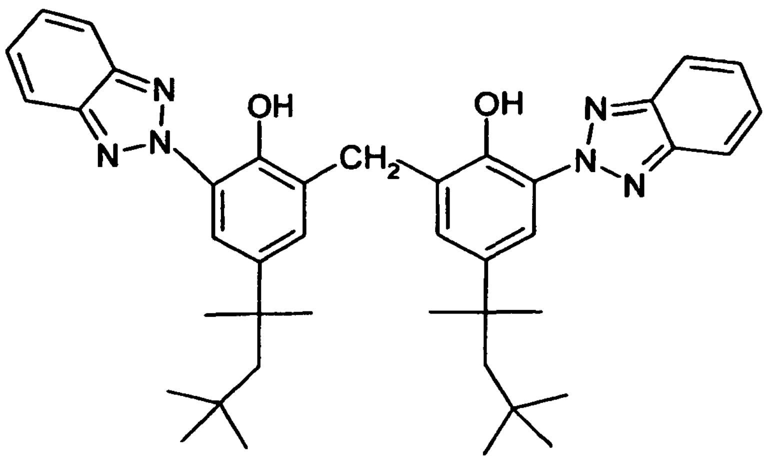

**composé (a)**

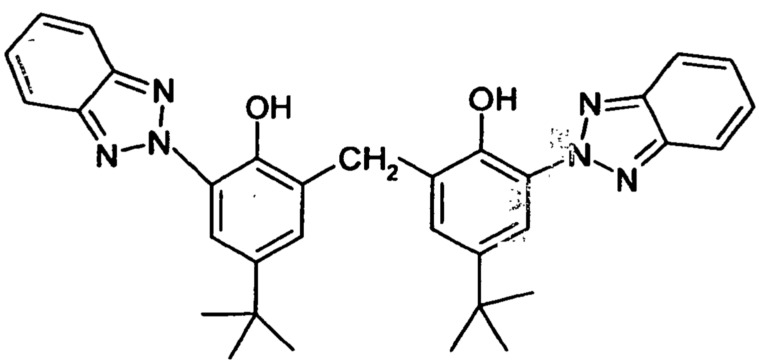

**composé (b)**

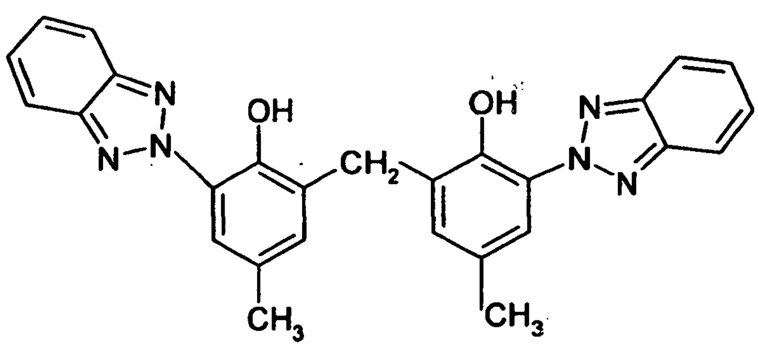

**composé (c)**

Le composé (a) de nomenclature 2,2'-méthylène-bis-[6-(2H-benzotriazol-2-yl)-4-(1,1,3,3-tetraméthylbutyl) phénol] est vendu sous le nom MIXXIM BB/100 par le société FAIRMOUNT CHEMICAL et sous forme micronisée sous le nom TINOSORB M par la société CIBA GEIGY.

Le composé (c) de nomenclature 2,2'-méthylène-bis-[6-(2H-benzotriazol-2-yl)-4-(methyl)phénol] est vendu sous le nom MIXXIM BB/200 par le société FAIRMOUNT CHEMICAL.

Parmi les filtres organiques insolubles du type amide vinylique, on peut citer par exemple les composés de formules suivante qui sont décrits dans la demande WO95/22959 :

$$T_{14}\text{-(Y)r-C(=O)-C(}T_{15}\text{)=C(}T_{16}\text{)-N(}T_{17}\text{)(}T_{18}\text{)} \quad (9)$$

dans laquelle $T_{14}$ est un radical alkyle en $C_1$-$C_{18}$, de préférence en $C_1$-$C_5$ ou un groupe phényle éventuellement substitué par un, deux ou trois radicaux choisis parmi OH, alkyle en $C_1$-$C_{18}$, alcoxy en $C_1$-$C_8$, ou un groupe -C(=O)-O$T_{19}$ où $T_{19}$ est un alkyle en $C_1$-$C_{18}$ ; $T_{15}$, $T_{16}$, $T_{17}$ et $T_{18}$ identiques ou différents désignent un radical alkyle en $C_1$-$C_{18}$, de préférence en $C_1$-$C_5$ ou un atome d'hydrogène ; Y est N ou O et r vaut 0 ou 1.

Parmi ces composés, on citera plus particulièrement :

- la 4-octylamino-3-pentèn-2-one ;
- l'éthyl-3-octylamino-2-buténoate ;
- la 3-octylamino-1-phényl-2-butèn-1-one
- la 3-dodecylamino-1-phenyl-2-buten-1-one.

Parmi les filtres organiques du type cinnamamide conformes à l'invention, on peut citer également les composés tels que décrits dans la demande WO95/22959 et répondant à la structure suivante :

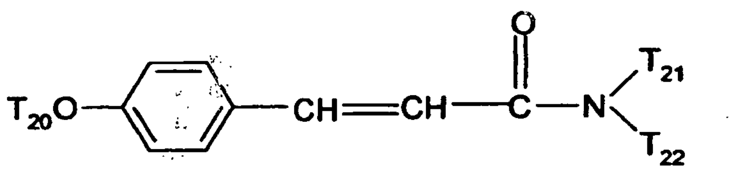

**(10)**

dans laquelle O$T_{20}$ est un radical hydroxy ou alcoxy en $C_1$-$C_4$, de préférence méthoxy ou éthoxy ; $T_{21}$ est hydrogène, alkyle en $C_1$-$C_4$, de préférence méthyle ou éthyle ; $T_{22}$ est un groupe -(CONH)s-phényle ou s vaut 0 ou 1 et le groupe phényle peut être substitué par un, deux ou trois groupes choisis parmi OH, alkyle en $C_1$-$C_{18}$, alcoxy en $C_1$-$C_8$, ou un groupe -C(=O)-O$T_{23}$ où $T_{23}$ est un alkyle en $C_1$-$C_{18}$ et plus préférentiellement $T_{23}$ est un groupe phényle, 4-méthoxyphényle ou phénylaminocarbonyle.

On peut également citer les dimères cinnamamides tels que ceux décrits dans le brevet US 5888481 comme par exemple le composé de structure :

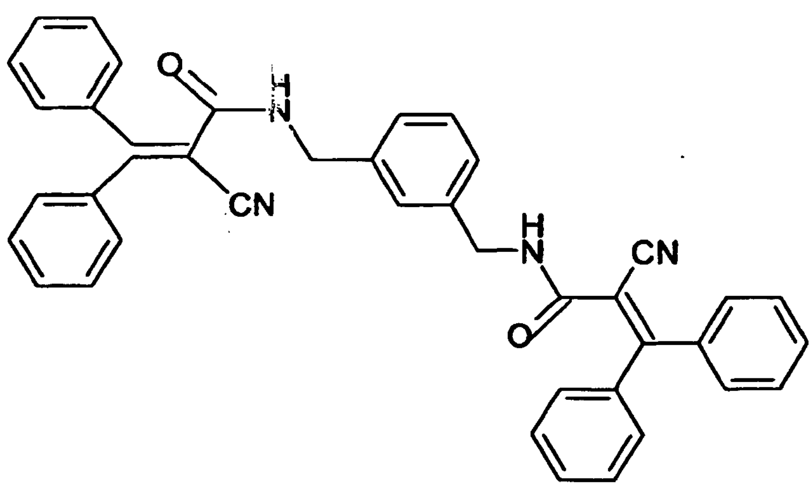

Une autre famille particulière de filtres organiques insolubles conformes à l'invention sont les sels de métaux polyvalents (par exemple $Ca^{2+}$, $Zn^{2+}$, $Mg^{2+}$, $Ba^{2+}$, $Al^{3+}$ ou $Zr^{4+}$) de filtres organiques sulfoniques ou carboxyliques tels que les sels de métaux polyvalents de dérivés sulfonés de benzylidène camphre tels que ceux décrits dans la demande FR-A 2 639 347 ; les sels de métaux polyvalents de dérivés sulfonés de benzimidazole tels que ceux décrits dans la demande EP-A-893 119 ; les sels de métaux polyvalents de dérivés d'acide cinnamique tels que ceux décrits dans la demande JP-87 166 517.

On peut également citer les complexes de métaux ou d'ammonium ou d'ammonium substitué de filtres organiques UV-A et/ou UV-B tels que décrits dans les demandes de brevet WO93/10753, WO93/11095 et WO95/05150.

Le ou les filtres organiques insolubles selon l'invention sont généralement présents dans les compositions filtrantes selon l'invention à une concentration totale comprise entre 0,1 et 15 % en poids environ, et de préférence entre 0,2 et 10 % en poids environ, par rapport au poids total de la composition.

Les compositions cosmétiques antisolaires selon l'invention peuvent bien entendu contenir un ou plusieurs filtres organiques complémentaires actifs dans l'UVA et/ou l'UVB (absorbeurs), solubles dans au moins l'une des phases des compositions. Ces filtres complémentaires peuvent être notamment choisis parmi les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469 et EP 933376 ; les dérivés de la benzophénone ; les dimères dérivés d'a-alkylstyrène tels que ceux décrits dans la demande DE 198 55 649 ; les dérivés de β,β'-diphénylacrylate, les dérivés de benzimidazole ; les dérivés bis-benzoazolyle tels que décrits dans les brevets EP-A-0669323 et US 2,463,264; les dérivés de l'acide p-aminobenzoïque ; les polymères filtres et silicones filtres tels que ceux décrits notamment dans la demande WO-93/04665.

Comme exemples de filtres solaires complémentaires actifs dans l'UV-A et/ou l'UV-B, solubles dans au moins l'une des phases des compositions, on peut citer :

l'acide p-aminobenzoïque,
le p-aminobenzoate oxyéthyléné (25mol),
le p-diméthylaminobenzoate de 2-éthylhexyle,
le p-aminobenzoate d'éthyle N-oxypropyléné
le p-aminobenzoate de glycérol,
le salicylate d'homomenthyle,
le salicylate de 2-éthylhexyle,
le salicylate de triéthanolamine,
le salicylate de 4-isopropylbenzyle,
le 4-ter-butyl-4'-méthoxy-dibenzoylméthane,
le 4-isopropyl-dibenzoylméthane,
le 4-méthoxy cinnamate de 2-éthylhexyle,
le diisopropyl cinnamate de méthyle,
le 4-méthoxy cinnamate d'isoamyle,
le 4-méthoxy cinnamate de diéthanolamine,
l'anthranilate de menthyle,
le 2-éthylhexyl-2-cyano-3,3'-diphénylacrylate,
l'éthyl-2-cyano-3,3'-diphénylacrylate,
l'acide 2-phényl benzimidazole 5-sulfonique et ses sels,
le 3-(4'-triméthylammonium)-benzylidèn-bornan-2-on-méthylsulfate,
le 2-hydroxy-4-méthoxybenzophénone,
le 2-hydroxy-4-méthoxybenzophénone-5-sulfonate,
le 2,4-dihydroxybenzophénone,
le 2,2',4,4'-tétrahydroxybenzophénone,
le 2,2'-dihydroxy-4,4'diméthoxybenzophénone,
le 2-hydroxy-4-n-octoxybenzophénone,
le 2-hydroxy-4-méthoxy-4'-méthylbenzophénone,
l'acide a-(2-oxoborn-3-ylidène)-tolyl-4-sulfonique et ses sels solubles
le 3-(4'-sulfo)benzylidèn-bornan-2-one et ses sels solubles,
le 3-(4'méthylbenzylidène)-d,l-camphre,
le 3-benzylidène-d,l-camphre,
l'acide benzène 1,4-di(3-méthylidène-10-camphosulfonique) et ses sels solubles,
l'acide urocanique,
la 2,4-bis {[4-2-éthyl-hexyloxy)]-2-hydroxy]-phenyl}-6-(4-méthoxy-phenyl)-1,3,5-triazine ;
le polymère de N-(2 et 4)-[(2-oxoborn-3-ylidèn)méthyl] benzyl]-acrylamide,
l'acide 1,4-bisbenzimidazolyl-phénylen-3,3',5,5'-tétrasulfonique et ses sels solubles.
les polyorganosiloxanes à fonction benzalmalonate
les polyorganosiloxanes à fonction benzotriazole tel que le Drometrizole Trisiloxane.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques selon l'invention peuvent encore contenir des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Les compositions de l'invention peuvent comprendre en outre des adjuvants cosmétiques classiques notamment choisis parmi les corps gras, les solvants organiques, les épaississants, les adoucissants, les opacifiants, les stabilisants, les émollients, les agents anti-mousse, les agents hydratants, les parfums, les conservateurs, les polymères, les charges, les séquestrants, les propulseurs, les agents alcalinisants ou acidifiants ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les polyoléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Bien entendu, l'homme de l'art veillera à choisir ce ou ces éventuels composés complémentaires et/ou leurs quantités de manière telle que les propriétés avantageuses, en particulier la rémanence à l'eau, la stabilité, attachées intrinsèquement aux émulsions conformes à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile.

Ces compositions peuvent se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

La phase aqueuse de celle-ci peut comprendre une dispersion vésiculaire non ionique préparée selon des procédés connus (Bangham, Standish and Watkins. J. Mol. Biol. 13, 238 (1965), FR2315991 et FR2416008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de poudre, de stick, de mousse aérosol ou de spray.

Lorsque la composition cosmétique selon l'invention est utilisée pour la protection des cheveux contre les rayons UV, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation .

Comme indiqué en début de description, un autre objet de la présente invention réside dans l'utilisation d'une émulsion selon l'invention pour la fabrication de compositions cosmétiques pour la protection de la peau et/ou des cheveux contre le rayonnement ultraviolet, en particulier le rayonnement solaire.

Un autre objet de la présente invention réside dans l'utilisation d'une émulsion sans émulsionnant comme support, pour la préparation d'une émulsion cosmétique ou dermatologique photoprotectrice, contenant au moins un filtre UV organique insoluble dans ladite émulsion tel que défini précédemment en vue d'améliorer la résistance à l'eau

de son pouvoir filtrant (rémanence à l'eau).

Des exemples concrets, mais nullement limitatifs, illustrant l'invention, vont maintenant être donnés.

| COMPOSITION | EX 1 |
|---|---|
| Benzoate d'alcools en C12/C15 (WITCONOL TN -WITCO) | 10g |
| Methylène bis-(tétraméthylbutyl hydroxyphényl benzotriazole) sous forme insoluble micronisée vendue sous le nom TINOSORB M par CIBA GEIGY- Taille moyenne de particule 0,15-0,2 µm | 2.5g |
| Octocrylène (UVINUL N539 - BASF) | 5g |
| 4-tertio-butyl-4'-méthoxydibenzoylméthanol (PARSOL 1789 - HOFFMANN LAROCHE) | 2g |
| Copolymère acide acrylique / acrylate d'alkyle ( C10 -C30 ) réticulé ( PEMULEN TR1 - GOODRICH ) | 0.75g |
| Oxyde de titane ( TITANIUM DIOXYDE MT100 TV - TAYCA ) | 3g |
| EDTA | 0.1g |
| Acide benzène 1,4-di(3-méthylidène-10-camphosulfonique) (MEXORYL SX-CHIMEX) | 0.5g |
| Glycérine | 5g |
| Triéthanolamine , conservateurs pH =7 | qs |
| Eau déminéralisée qsp | 100 g |

| COMPOSITION | EX 2 |
|---|---|
| Benzoate d'alcools en C12/C15 (WITCONOL TN -WITCO) | 8g |
| Methylène bis-(tétraméthylbutyl hydroxyphényl benzotriazole) sous forme insoluble micronisée vendue sous le nom TINOSORB M par CIBA GEIGY- Taille moyenne de particule 0,15-0,2 µm | 3g |
| Octocrylène (UVINUL N539-BASF) | 5g |
| 2,4-bis{[4-2-éthyl-hexyloxy)]-2-hydroxy]-phényl}-6-(4-méthoxy-phényl)-1,3,5-triazine 2., 4-bis{[4-2-éthyl-héxyloxy)] | 3g |
| Terpolymère acide méthacrylique/acrylate d'éthyle/stéareth10 allyl ether reticulé en émulsion aqueuse (SALCARE-SC90-ALLIED COLLOIDS) | 5g |
| Oxyde de titane ( TITANIUM DIOXYDE MT100 TV - TAYCA ) | 3g |
| Glycérine | 5g |
| Triéthanolamine , conservateurs | qs pH =7 |
| Eau déminéralisée qsp | 100 g |

## Revendications

**1.** Emulsion cosmétique ou dermatologique sans émulsionnant, **caractérisée par le fait qu'**elle comporte:

a) au moins une phase aqueuse et
b) au moins une phase grasse ;
c) au moins un système photoprotecteur capable de filtrer les rayons UV contenant au moins filtre UV organique insoluble dans ladite émulsion, sous forme micronisée avec une taille moyenne de particule allant de 0,01 à 2µm.
d) au moins un polymère réticulé d'au moins un monomère à insaturation éthylénique et
e) éventuellement un tensio-actif permettant d'améliorer la dispersion des particules de filtre insoluble lorsque celui-ci est obtenu par broyage d'un filtre UV insoluble sous forme de particules de taille grossière en présence dudit tensio-actif.

**2.** Emulsion selon la revendication 1, où le ou les filtres UV insolubles sous forme insoluble micronisée a une taille

moyenne de particule allant de 0,02 à 1,5 μm.

3. Emulsion selon la revendication 2, où le ou les filtres UV insolubles sous forme insoluble micronisée a une taille moyenne de particule allant de 0,03 à 1,0 μm.

4. Emulsion selon l'une quelconque des revendications 1 à 3, **caractérisé par le fait que** le ou les filtres UV insolubles sous forme micronisée sont susceptibles d'être obtenus par un procédé de broyage du filtre organique insoluble sous forme de particules de taille grossière en présence d'un tensio-actif permettant d'améliorer la dispersion des particules de filtre insoluble.

5. Emulsion selon la revendication 1 à 4, où le tensio-actif est choisi parmi les alkylpolyglucosides de structure $C_nH_{2n+1}O(C_6H_{10}O_5)_xH$ dans laquelle n est un entier de 8 à 16 et x est le degré moyen de polymérisation de l'unité $(C_6H_{10}O_5)$ et varie de 1,4 à 1,6.

6. Emulsion selon l'une quelconque des revendications 1 à 5, **caractérisé par le fait que** le tensio-actif est utilisé à une concentration allant de 1 à 50% en poids par rapport au filtre UV organique insoluble dans sa forme micronisée.

7. 8 Emulsion selon la revendication 1 à 6, où le polymère réticulé d'au moins un monomère à insaturation éthylénique, est choisi dans le groupe constitué par :

   (i) les homopolymères ou copolymères réticulés d'au moins un monomère à insaturation éthylénique à fonction sulfonique, sous forme libre ou partiellement ou totalement salifiée ;
   (ii) les copolymères réticulés d'au moins un monomère à insaturation éthylénique à fonction acide carboxylique, sous forme libre ou partiellement ou totalement salifiée ; ou d'un dérivé ester ou amide,
   (iii) les homopolymères ou copolymères réticulés d'au moins un monomère à insaturation éthylénique cationique du type ester ou amide ;

8. Emulsion selon la revendication 7, où les homopolymères ou copolymères réticulés d'au moins un monomère à insaturation éthylénique à fonction sulfonique sont choisis dans le groupe constitué par :

   - les homopolymères réticulés d'acide 2-acrylamido-2-méthyl-propane sulfonique, neutralisés à au moins 90% ;
   - les copolymères réticulés d'acide 2-acrylamido-2-méthyl-propane sulfonique et d'acrylamide partiellement ou totalement neutralisés.

9. Emulsion selon la revendication 7, où les copolymères réticulés d'au moins un monomère à insaturation éthylénique à fonction acide carboxylique ou d'un dérivé ester ou amide, sont choisis parmi les polymères amphiphiles non-ioniques ou anioniques comportant au moins une chaîne grasse et au moins un motif hydrophile.

10. Emulsion selon la revendication 9, où les polymères amphiphiles non-ioniques sont choisis parmi :

   (a) les copolymères de méthacrylates ou d'acrylates d'alkyles en $C_1$-$C_6$ et de monomères amphiphiles comportant au moins une chaîne grasse ;
   (b) les copolymères de méthacrylates ou d'acrylates hydrophiles et de monomères hydrophobes comportant au moins une chaîne grasse.

11. Emulsion selon la revendication 9, où les polymères amphiphiles anioniques sont choisis parmi ceux dont le motif hydrophile est constitué par un monomère anionique insaturé éthylénique, plus particulièrement par un acide carboxylique vinylique.

12. Emulsion selon la revendication 11, où les polymères amphiphiles anioniques sont choisis parmi ceux dont le motif hydrophile est constitué par un acide acrylique, un acide méthacrylique ou leurs mélanges, et dont le motif hydrophobe à chaîne grasse correspond au monomère de formule (I) suivante :

$$CH_2 = C(R')\ CH_2\ O\ B_n\ R \qquad (I)$$

dans laquelle R' désigne H ou $CH_3$, B désigne le radical éthylèneoxy, n est nul ou désigne un entier allant de 1 à

100, R désigne un radical hydrocarboné choisi parmi les radicaux alkyl, arylalkyl, aryl, alkylaryl, cycloalkyl, comprenant 8 à 30 atomes de carbone, de préférence 10 à 24, et plus particulièrement encore de 12 à 18 atomes de carbone.

**13.** Emulsion selon la revendication 12, où dans la formule (I) : R' désigne H, n est égal à 10, et R désigne un radical stéaryle ($C_{18}$).

**14.** Emulsion selon la revendication 12 ou 13, où les polymères amphiphiles anioniques sont formés à partir de 20 à 60% en poids d'acide acrylique et/ou d'acide méthacrylique, de 5 à 60% en poids de (méth)acrylates d'alkyle inférieurs, de 2 à 50% en poids d'éther d'allyle à chaîne grasse de formule (I), et de 0 à 1% en poids d'un agent réticulant.

**15.** Emulsion selon la revendication 12 ou 13, où les polymères amphiphiles anioniques sont choisis parmi les terpolymères réticulés d'acide méthacrylique, d'acrylate d'éthyle, de polyéthylèneglycol (10 OE) éther d'alcool stéarylique (Steareth 10).

**16.** Emulsion selon la revendication 13, où les polymères amphiphiles anioniques sont choisis parmi ceux comportant au moins un motif hydrophile de type acide carboxylique insaturé oléfinique, et au moins un motif hydrophobe de type ester d'alkyl ($C_{10}$-$C_{30}$) d'acide carboxylique insaturé.

**17.** Emulsion selon la revendication 16, où les polymères amphiphiles sont choisis de préférence parmi ceux dont le motif hydrophile de type acide carboxylique insaturé oléfinique correspond au monomère de formule (II) suivante :

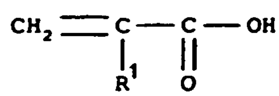

(II)

formule dans laquelle, $R^1$ désigne H ou $CH_3$ ou $C_2H_5$, c'est-à-dire des motifs acide acrylique, acide méthacrylique ou acide éthacrylique et dont le motif hydrophobe de type ester d'alkyl ($C_{10}$-$C_{30}$) d'acide carboxylique insaturé correspond au monomère de formule (III) suivante :

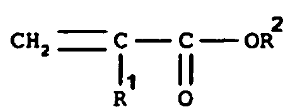

(III)

formule dans laquelle, $R^1$ désigne H ou $CH_3$ ou $C_2H_5$ (c'est-à-dire des motifs acrylates, méthacrylates ou éthacrylates) et de préférence H (motifs acrylates) ou $CH_3$ (motifs méthacrylates), $R^2$ désignant un radical alkyle en $C_{10}$-$C_{30}$, et de préférence en $C_{12}$-$C_{22}$.

**18.** Emulsion selon la revendication 7, où les homopolymères ou copolymères réticulés d'au moins un monomère à insaturation éthylénique cationique du type ester ou amide sont choisis parmi :

(1) les homopolymères d'acrylate d'ammonium ou les copolymères d'acrylate d'ammonium et d'acrylamide;
(2) les homopolymères de diméthylaminoéthyl-méthacrylate quaternisé par le chlorure de méthyle ou les copolymères de diméthylamino-éthylméthacrylate quaternisé par le chlorure de méthyle.

**19.** Emulsion selon l'une quelconque des revendications 1 à 18, dans laquelle le ou les polymères réticulés sont généralement présents à une concentration allant de 0,01 à 10 % en poids, et de préférence de 0,1 à 5 % en poids, par rapport au poids total de la composition.

**20.** Emulsion selon l'une quelconque des revendications 1 à 19, où le ou les filtres UV organiques insolubles sous forme micronisée sont choisis parmi les filtres UV organiques insolubles du type oxanilide, du type triazine, du type triazole, du type amide vinylique ou du type cinnamide

**21.** Emulsion selon la revendication 20, où les filtres UV du type oxaniliden répondent à la structure :

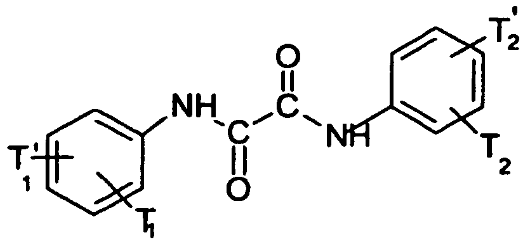

(1)

dans laquelle $T_1$, $T'_1$, $T_2$ et $T'_2$ désignent, identiques et différents, un radical alkyle en $C_1$-$C_8$ ou un radical alcoxy en $C_1$-$C_8$ .

**22.** Emulsion selon la revendication 20, où les filtres UV du type triazine sont choisis parmi ceux répondant à la formule générale suivante :

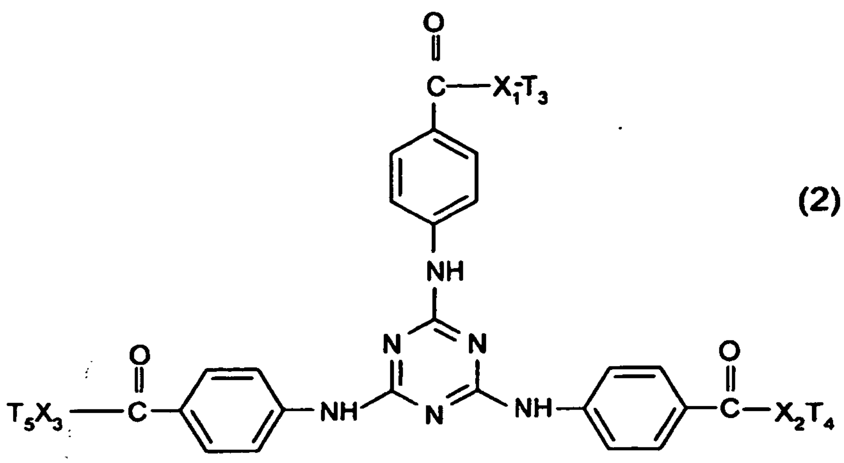

(2)

dans laquelle :

- $X_1$, $X_2$ et $X_3$, identiques ou différents, représentent l'oxygène ou un radical -NZ-;
- les radicaux Z, identiques ou différents, désignent hydrogène ou un radical alkyle en $C_1$-$C_{18}$, linéaire ou ramifié, un radical cycloalkyle en $C_5$-$C_{12}$ pouvant être substitué par un ou plusieurs radicaux alkyle en $C_1$-$C_4$ ;
- $T_3$, $T_4$ et $T_5$, identiques ou différents, sont choisis parmi : l'hydrogène; un métal alcalin; un radical ammonium éventuellement substitué par un ou plusieurs radicaux alkyle ou hydroxyalkyle ; un radical alkyle linéaire ou ramifié en $C_1$-$C_{18}$ ; un radical cycloalkyle en $C_5$-$C_{12}$ éventuellement substitué par un ou plusieurs radicaux alkyle en $C_1$-$C_4$ ; un radical polyoxyéthyléné comprenant de 1 à 6 unités d'oxyde d'éthylène et dont le groupe OH terminal est méthylé ; un radical de formule (3), (4) ou (5) suivantes :

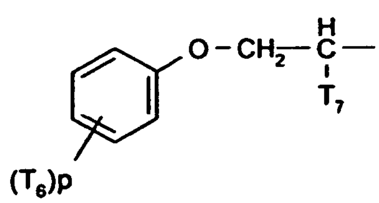

(3)

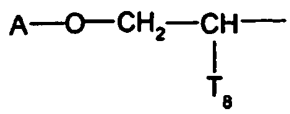 (4)

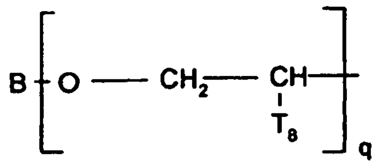 (5)

dans lesquelles :

- $T_6$ est l'hydrogène ou un radical méthyle ;
- $T_7$ est un radical alkyle en $C_1$-$C_9$ ;
- p est un nombre entier allant de 0 à 3 ;
- q est un nombre entier allant de 1 à 10 ;
- A est un radical alkyle en $C_4$-$C_8$ ou un radical cycloalkyle en $C_5$-$C_8$ ;
- B est choisi parmi : un radical alkyle linéaire ou ramifié en $C_1$-$C_8$ ; un radical cycloalkyle en $C_5$-$C_8$ ; un radical aryle éventuellement substitué par un ou plusieurs radicaux alkyle en $C_1$-$C_4$ ;
- $T_8$ est l'hydrogène ou un radical méthyle.

**23.** Emulsion selon la revendication 22, où le filtre UV du type triazine répond à la formule suivante :

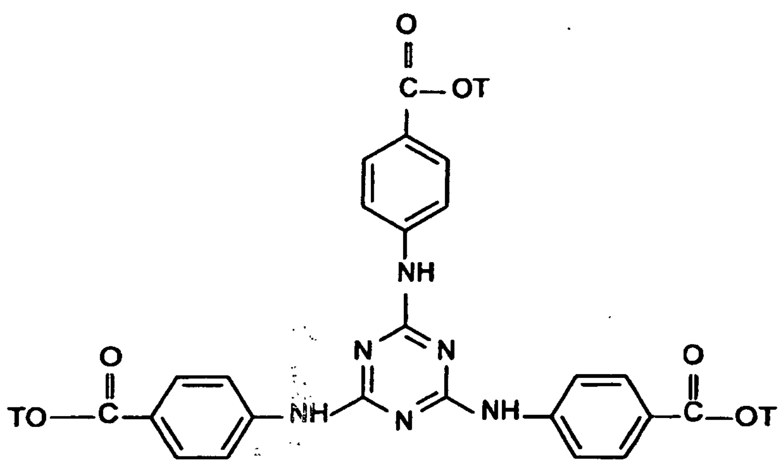

dans laquelle T désigne un radical 2-éthylhexyle ;

**24.** Emulsion selon la revendication 22, où le filtre UV du type triazine répond à la formule suivante :

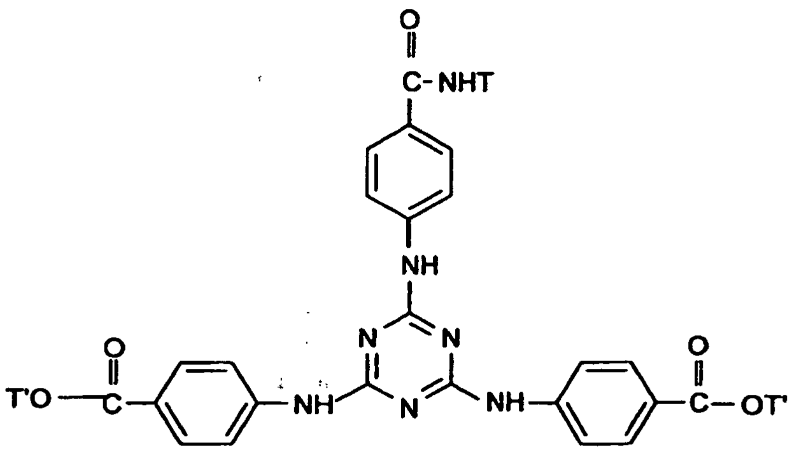

dans laquelle T' désigne un radical 2-éthylhexyle et T désigne un radical tert-butyle.

**25.** Emulsion selon la revendication 20 , où les filtres UV du type triazine sont choisis parmi les dérivés insolubles de s-triazine portant des groupements benzalmalonates et/ou phenylcyanoacrylates .

**26.** Emulsion selon la revendication 25, où les filtres UV du type triazine sont choisis parmi les composés suivants :

- la 2,4,6-tris(4'-amino benzalmalonate de diéthyle)-s-triazine,
- la 2,4,6-tris(4'-amino benzalmalonate de diisopropyle)-s-triazine,
- la 2,4,6-tris(4'-amino benzalmalonate de diméthyle)-s-triazine,
- la 2,4,6-tris($\alpha$-cyano-4-aminocinnamate d'éthyle)-s-triazine.

**27.** Emulsion selon la revendication 20 , où les filtres UV du type triazine sont choisis parmi ceux répondant à la formule suivante :

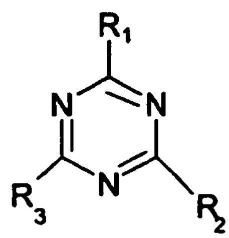

**(6)**

dans laquelle $R_1$, $R_2$, $R_3$,, indépendamment, sont phenyle, phenoxy, pyrrolo, dans lesquels les phenyle, phenoxy, pyrrolo sont éventuellement substitués par un, deux ou trois substituants choisis parmi OH, $C_1$-$C_{18}$alkyle ou alkoxy, $C_1$-$C_{18}$carboxyalkyle, $C_5$-$C_8$cycloalkyle, un groupe méthylidènecamphre, un groupe -$(CH=CH)_n(CO)$-$OR_4$ , avec $R_4$ soit $C_1$-$C_{18}$alkyle soit cinnamyle, et n vaut 0 ou 1.

**28.** Emulsion selon la revendication 20, où les filtres UV du type triazine sont choisis parmi les dérivés insolubles de s-triazine portant des groupements benzotriazoles et/ou benzothiazoles .

**29.** Emulsion selon la revendication 28, où les filtres UV du type triazine sont choisis parmi

- la 2,4,6-tris[(3'-benzotriazol-2-yl-2'-hydroxy-5'-methyl) phenylamino]-s-triazine,
- 2,4,6-tris[(3'-benzotriazol-2-yl-2'-hydroxy-5'-ter-octyl) phénylamino]-s-triazine.

**30.** Emulsion selon la revendication 20, où les filtres UV organiques du type triazole répondent à la formule (7) suivante :

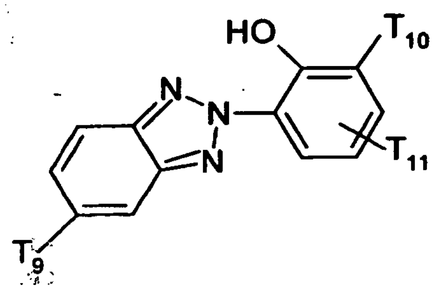

(7)

dans laquelle $T_9$ désigne un atome d'hydrogène ou un radical alkyle en $C_1$-$C_{18}$ ; $T_{10}$ et $T_{11}$, identiques ou différents, désignent un radical alkyle en $C_1$-$C_{18}$ éventuellement substitué par un phényle.

**31.** Emulsion selon la revendication 30, où le composé de formule (7) est choisi parmi les composés suivants :

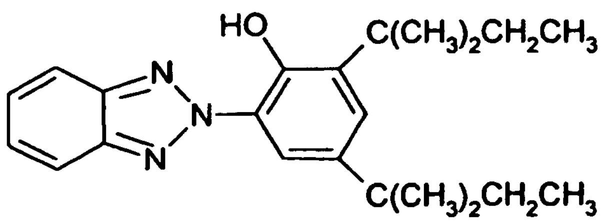

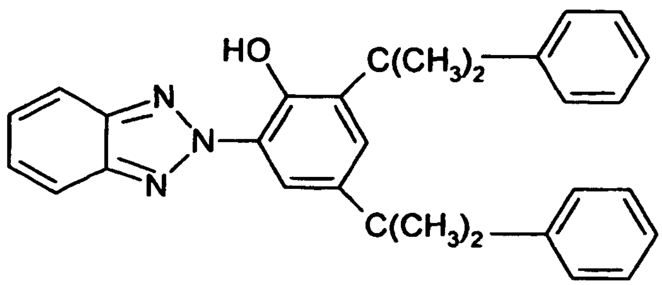

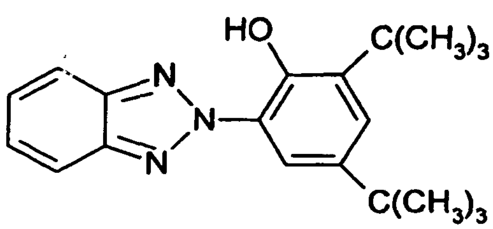

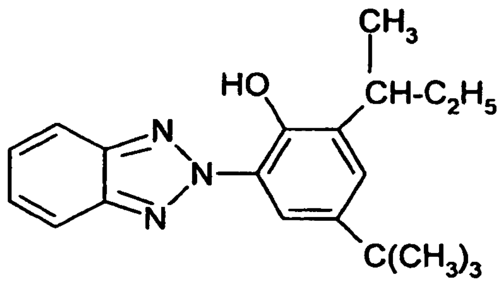

**32.** Emulsion selon la revendication 20, où le filtre UV insoluble est le [2 ,4'-dihydroxy-3-(2H-benzotriazol-2-yl)-5-(1,1,3,3-tétraméthylbutyl)-2'-n-octoxy-5'-benzoyl] diphénylméthane de structure :

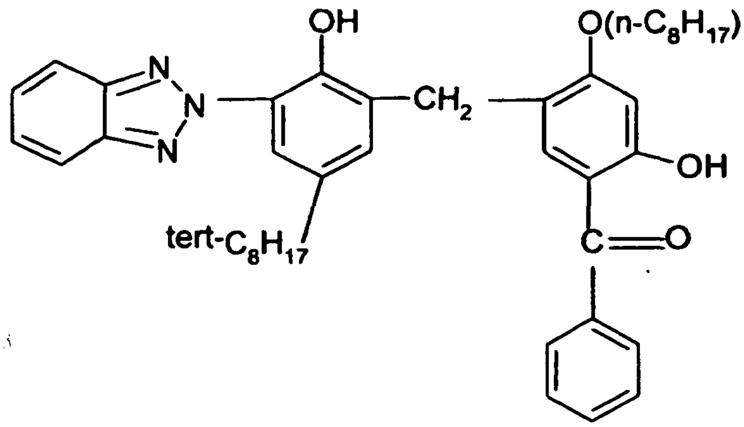

**33.** Emulsion selon la revendication 20 où les filtres UV organiques du type triazole sont choisis parmi les dérivés de méthylène bis-(hydroxyphényl benzotriazole) de structure suivante :

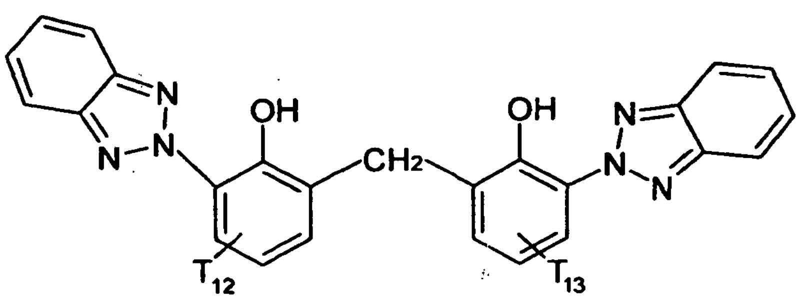

**(8)**

dans laquelle les radicaux $T_{12}$ et $T_{13}$, identiques ou différents, désignent un radical alkyle en $C_1$-$C_{18}$ pouvant être substitué par un ou plusieurs radicaux choisis parmi alkyle en $C_1$-$C_4$, cycloalkyle en $C_5$-$C_{12}$ ou un reste aryle.

**34.** Emulsion selon la revendication 33, où le composé de formule (8) est choisi dans le groupe constitué par les composés de structure suivante :

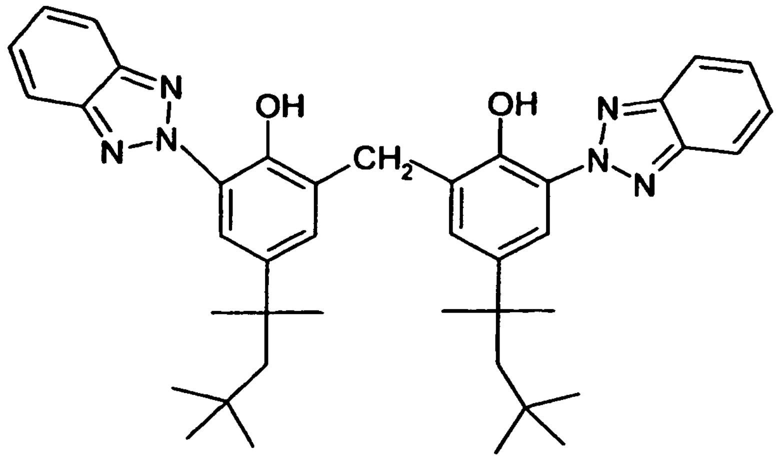

composé (a)

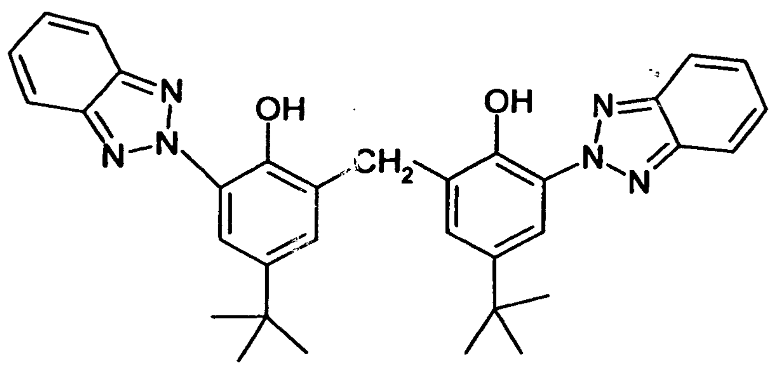

composé (b)

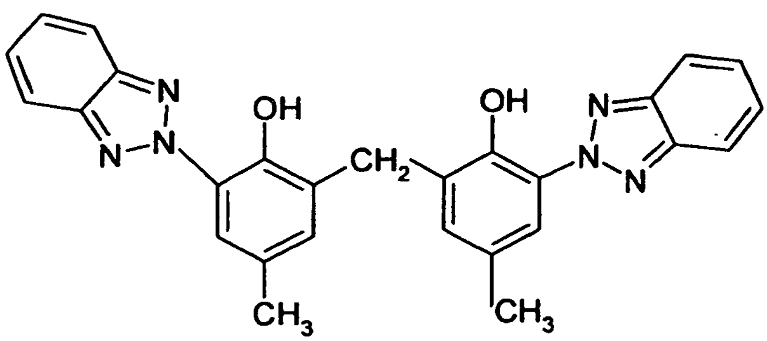

composé (c)

**35.** Emulsion selon la revendication 20, où les filtres organiques du type amide vinylique, répondent à la formule suivante :

$$T_{14}\text{-}(Y)r\text{-}C(=O)\text{-}C(T_{15})=C(T_{16})\text{-}N(T_{17})(T_{18}) \qquad (9)$$

dans laquelle $T_{14}$ est un radical alkyle en $C_1$-$C_{18}$, de préférence en $C_1$-$C_5$ ou un groupe phényle éventuellement substitué par un, deux ou trois radicaux choisis parmi OH, alkyle en $C_1$-$C_{18}$, alcoxy en $C_1$-$C_8$, ou un groupe -C(=O)-O$T_{19}$ où $T_{19}$ est un alkyle en $C_1$-$C_{18}$ ; $T_{15}$, $T_{16}$, $T_{17}$ et $T_{18}$ identiques ou différents désignent un radical alkyle en $C_1$-$C_{18}$, de préférence en $C_1$-$C_5$ ou un atome d'hydrogène ; Y est N ou O et r vaut 0 ou 1.

**36.** Emulsion selon la revendication 35, où les composés de formule (9) sont choisis parmi :

- la 4-octylamino-3-pentèn-2-one ;
- l'éthyl-3-octylamino-2-buténoate ;
- la 3-octylamino-1-phényl-2-butèn-1-one
- la 3-dodecylamino-1-phenyl-2-buten-1-one.

**37.** Emulsion selon la revendication 20, où les filtres organiques du type cinnamamide répondent à la formule suivante :

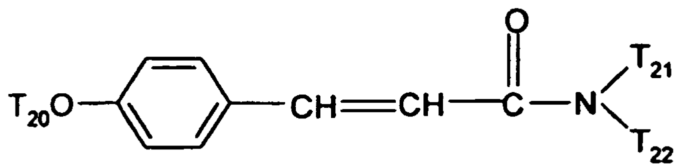

(10)

dans laquelle $OT_{20}$ est un radical hydroxy ou alcoxy en $C_1$-$C_4$, de préférence méthoxy ou éthoxy ; $T_{21}$ est hydrogène, alkyle en $C_1$-$C_4$, de préférence méthyle ou éthyle ; $T_{22}$ est un groupe -(CONH)s-phényle ou s vaut 0 ou 1 et le groupe phényle peut être substitué par un, deux ou trois groupes choisis parmi OH, alkyle en $C_1$-$C_{18}$, alcoxy en $C_1$-$C_8$, ou un groupe -C(=O)-$OT_{23}$ où $T_{23}$ est un alkyle en $C_1$-$C_{18}$ et plus préférentiellement $T_{23}$ est un groupe phényle, 4-méthoxyphényle ou phénylaminocarbonyle.

**38.** Emulsion selon la revendication 20, où le filtre UV insoluble est un dimère cinnamamide.

**39.** Emulsion selon la revendication 38, où le filtre UV insoluble est le composé de structure :

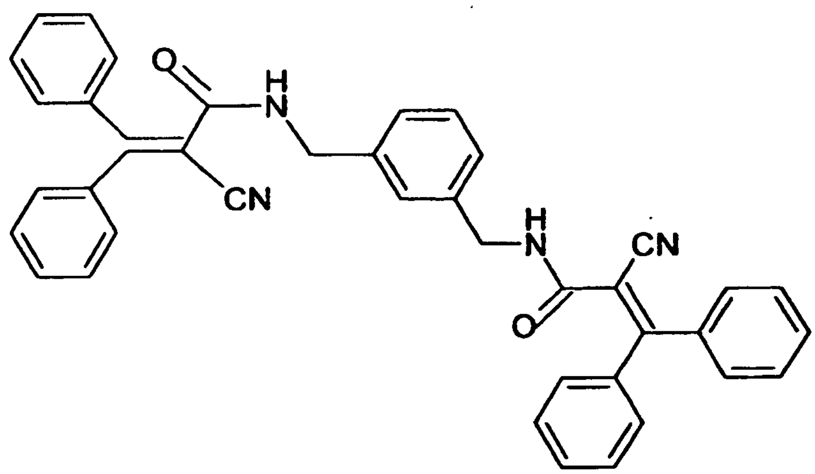

**40.** Emulsion selon l'une quelconque des revendications 1 à 19, où les filtres UV insolubles sont des sels de métaux polyvalents de filtres UV organiques sulfoniques ou carboxyliques.

**41.** Emulsion selon la revendication 40 , où les filtres UV insolubles sont des sels de métaux polyvalents de dérivés sulfonés de benzylidène camphre ; les sels de métaux polyvalents de dérivés sulfonés de benzimidazole ; les sels de métaux polyvalents de dérivés d'acide cinnamique.

**42.** Emulsion selon l'une quelconque des revendications 1 à 19, où les filtres UV insolubles sont des complexes de métaux polyvalents ou d'ammonium ou d'ammonium substitué de filtres organiques UV-A et/ou UV-B

**43.** Emulsion selon l'une quelconque des revendications précédentes, **caractérisée par le fait qu'**elle comprend en outre un ou plusieurs filtres organiques complémentaires actifs dans l'UV-A et/ou UV-B, solubles dans au moins l'une des phases de l'émulsion.

**44.** Emulsion selon la revendication 43, **caractérisée par le fait que** lesdits filtres organiques complémentaires sont

choisis parmi les dérivés cinnamiques ; les dérivés salicyliques, les dérivés du camphre; les dérivés de triazine ; les dérivés de dibenzoylméthane ; les dérivés de la benzophénone ; les dérivés de β,β'-diphénylacrylate, les dérivés de benzimidazole ; les dimères dérivés d'α-alkylstyrène ; les dérivés bis-benzoazolyle ; les dérivés de l'acide p-aminobenzoïque ; les polymères filtres et silicones filtres.

**45.** Emulsion selon l'une quelconque des revendications précédentes, **caractérisée par le fait qu'**elle comprend en outre, des pigments ou des nanopigments d'oxydes métalliques, enrobés ou non.

**46.** Emulsion selon la revendication 45, **caractérisée par le fait que** lesdits pigments ou nanopigments sont choisis parmi les oxydes de titane, les oxydes de zinc, les oxydes de fer, les oxydes de zirconium, les oxydes de cérium et leurs mélanges, enrobés ou non.

**47.** Emulsion selon l'une quelconque des revendications précédentes, **caractérisée par le fait qu'**elle comprend en outre au moins un agent de bronzage et/ou de brunissage artificiel de la peau.

**48.** Emulsion selon l'une quelconque des revendications précédentes 44 à 49, **caractérisée par le fait qu'**elle comprend en outre au moins un adjuvant choisi parmi les corps gras, les solvants organiques, les épaississants, les adoucissants, les opacifiants, les stabilisants, les émollients, les agents anti-mousse, les agents hydratants, les parfums, les conservateurs, les polymères, les séquestrants, les propulseurs, les agents alcalinisants ou acidifiants.

**49.** Emulsion selon l'une quelconque des revendications 1 à 48, **caractérisée par le fait qu'**il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une poudre, d'un bâtonnet solide, d'une mousse ou d'un spray.

**50.** Emulsion selon l'une quelconque des revendications 1 à 48, **caractérisée par le fait qu'**il s'agit d'une composition de maquillage des cils, des sourcils ou de la peau et qu'elle se présente sous forme solide ou pâteuse, anhydre ou aqueuse, d'une émulsion, d'une suspension ou d'une dispersion.

**51.** Emulsion selon l'une quelconque des revendications 1 à 48, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

**52.** Utilisation de l'émulsion définie dans l'une quelconque des revendications 1 à 42 pour la fabrication de compositions cosmétiques pour la protection de la peau et/ou des cheveux contre le rayonnement ultraviolet, en particulier le rayonnement solaire.

**53.** Utilisation d'une émulsion sans émulsionnant telle que définie selon l'une quelconque des revendications 1 à 42 comme support pour la préparation d'une émulsion cosmétique ou dermatologique photoprotectrice contenant au moins un filtre UV insoluble tel que défini dans l'une des revendications 1 à 42, en vue d'améliorer la résistance à l'eau de son pouvoir filtrant (rémanence à l'eau).

## Patentansprüche

**1.** Kosmetische oder dermatologische Emulsion ohne Emulgator, **dadurch gekennzeichnet, dass** sie enthält:

(a) mindestens eine wässrige Phase,
(b) mindestens eine Fettphase,
(c) mindestens ein Lichtschutzsystem, das befähigt ist, die UV-Strahlung zu filtern, und das mindestens ein in der Emulsion unlösliches, organisches UV-Filter in mikronisierter Form enthält, wobei die mittlere Größe der Partikel im Bereich von 0,01 bis 2 µm liegt,
(d) mindestens ein vernetztes Polymer mindestens eines Monomers mit ethylenischer Doppelbindung, und
(e) gegebenenfalls einen grenzflächenaktiven Stoff zur Verbesserung die Dispersion der Partikel eines unlöslichen Filters, wenn dieses durch Zerkleinern eines unlöslichen UV-Filters, das in Form von groben Partikeln vorliegt, in Gegenwart des grenzflächenaktiven Stoffes hergestellt wird.

**2.** Emulsion nach Anspruch 1, wobei das oder die unlösliche(n) UV-Filter, die in unlöslicher, mikronisierter Form vorliegen, eine mittlere Größe der Partikel im Bereich von 0,02 bis 1,5 µm aufweisen.

**3.** Emulsion nach Anspruch 2, wobei das oder die unlösliche(n) UV-Filter, die in unlöslicher, mikronisierter Form vorliegen, eine mittlere Größe der Partikel im Bereich von 0,03 bis 1,0 µm aufweisen.

**4.** Emulsion nach einem der Ansprüche 1 bis 3, **dadurch gekennzeichnet, dass** das oder die unlösliche(n) UV-Filter in mikronisierter Form durch Zerkleinern eines unlöslichen organischen Filters, das in Form von groben Partikel vorliegt, in Gegenwart eines grenzflächenaktiven Stoffes hergestellt werden kann, der die Dispersion der Partikel des unlöslichen Filters verbessern kann.

**5.** Emulsion nach Anspruch 1 bis 4, wobei der grenzflächenaktive Stoff unter den Alkylpolyglucosiden der Struktur $C_nH_{2n+1}O(C_6H_{10}O_5)_xH$ ausgewählt ist, worin n eine ganze Zahl von 8 bis 16 bedeutet und x der mittlere Polymerisationsgrad der Einheit ($C_6H_{10}O_5$) ist und im Bereich von 1,4 bis 1,6 liegt.

**6.** Emulsion nach einem der Ansprüche 1 bis 5, **dadurch gekennzeichnet, dass** der grenzflächenaktive Stoff in einer Konzentration von 1 bis 50 Gew.-%, bezogen auf das in mikronisierter Form vorliegende, unlösliche organische Filter verwendet wird.

**7.** Emulsion nach Anspruch 1 bis 6, wobei das vernetzte Polymer mindestens eines Monomers mit ethylenischer Doppelbindung unter den folgenden Polymeren ausgewählt ist:

(i) vernetzten Homopolymeren oder Copolymeren mindestens eines Monomers mit ethylenischer Doppelbindung und Sulfonsäurefunktion, die in freier Form vorliegt oder ganz oder teilweise in das Salz übergeführt ist;
(ii) vernetzten Copolymeren mindestens eines Monomers mit ethylenischer Doppelbindung und Carbonsäurefunktion, die in freier Form vorliegt oder ganz oder teilweise in das Salz übergeführt ist, oder eines Ester- oder Amidderivats;
(üi) vernetzten Homopolymeren oder Copolymeren mindestens eines kationischen Monomers mit ethylenischer Doppelbindung vom Ester- oder Amidtyp.

**8.** Emulsion nach Anspruch 7, wobei die vernetzten Homopolymere oder Copolymere mindestens eines Monomers mit ethylenischer Doppelbindung und Sulfonsäurefunktion unter den folgenden Polymeren ausgewählt sind:

- vernetzten Homopolymeren von 2-Acrylamido-2-methylpropansulfonsäure, die mindestens zu 90 % neutralisiert sind;
- vernetzten Copolymeren von 2-Acrylamido-2-methylpropansulfonsäure und Acrylamid, die ganz oder zum Teil neutralisiert sind.

**9.** Emulsion nach Anspruch 7, wobei die vernetzten Copolymere mindestens eines Monomers mit ethylenischer Doppelbindung und Carbonsäurefunktion oder eines Ester- oder Amidderivats unter den nichtionischen oder anionischen amphiphilen Polymeren ausgewählt sind, die mindestens eine Fettkette und mindestens eine hydrophile Einheit enthalten

**10.** Emulsion nach Anspruch 9, wobei die nichtionischen amphiphilen Polymere unter den folgenden Polymeren ausgewählt sind:

(a) Copolymeren von $C_{1-6}$-Alkylmethacrylaten oder -acrylaten und amphiphilen Monomeren, die mindestens eine Fettkette enthalten;
(b) Copolymeren von hydrophilen Methacrylaten oder Acrylaten und hydrophoben Monomeren, die mindestens eine Fettkette enthalten.

**11.** Emulsion nach Anspruch 9, wobei die anionischen amphiphilen Polymere unter den Polymeren ausgewählt sind, deren hydrophile Einheit aus mindestens einem anionischen Monomer mit ethylensicher Doppelbindung und insbesondere aus einer Vinylcarbonsäure besteht

**12.** Emulsion nach Anspruch 11, wobei die anionischen amphiphilen Polymere unter den Polymeren ausgewählt sind, deren hydrophile Einheit aus Acrylsäure, Methacrylsäure oder deren Gemischen besteht und deren hydrophobe Einheit mit Fettkette dem Monomer der folgenden Formel (I) entspricht:

$$CH_2{=}C(R')CH_2\ O\ B_n\ R \qquad (I),$$

worin bedeuten: R' H oder $CH_3$, B Ethylenoxy, n Null oder eine ganze Zahl von 1 bis 100, R eine Kohlenwasserstoffgruppe, die unter Alkyl, Arylalkyl, Aryl, Alkylaryl oder Cycloalkyl mit 8 bis 30 Kohlenstoffatomen, vorzugsweise 10 bis 24 Kohlenstoffatomen und insbesondere 12 bis 18 Kohlenstoffatomen ausgewählt ist.

**13.** Emulsion nach Anspruch 12, wobei die in der Formel (I) R' H, n 10 und R Stearyl ($C_{18}$) bedeutet.

**14.** Emulsion nach Anspruch 12 oder 13, wobei die anionischen amphiphilen Polymere aus 20 bis 60 Gew.-% Acrylsäure und/oder Methacrylsäure, 5 bis 60 Gew.-% Alkyl(meth)acrylaten, wobei es sich um niedere Alkylgruppen handelt, 2 bis 50 Gew.-% Allylether mit Fettkette der Formel (I) und 0 bis 1 Gew.-% Vernetzungsmittel gebildet werden.

**15.** Emulsion nach Anspruch 12 oder 13, wobei die anionischen amphiphilen Polymere unter den vernetzten Terpolymeren von Methacrylsäure, Ethylacrylat und dem Polyethylenglykolether (10 EO) von Stearylalkohol (Steareth-10) ausgewählt sind.

**16.** Emulsion nach Anspruch 13, wobei das anionische amphiphile Polymer unter den anionischen Polymeren ausgewählt ist, die mindestens eine hydrophile Einheit vom Typ einer olefinisch ungesättigten Carbonsäure und mindestens eine hydrophobe Einheit vom Typ eines $C_{10-30}$-Alkylesters einer ungesättigten Carbonsäure aufweisen.

**17.** Emulsion nach Anspruch 16, wobei die amphiphilen Polymere unter den Polymeren ausgewählt sind, deren hydrophile Einheit vom Typ einer olefinisch ungesättigten Carbonsäure einem Monomer der folgenden Formel (II) entspricht:

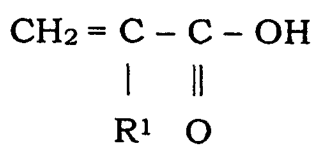

(II),

worin $R^1$ H oder $CH_3$ oder $C_2H_5$ bedeutet, d.h., es handelt sich um Acrylsäure-, Methacrylsäure- oder Ethacrylsäureeinheiten, und wobei die hydrophobe Einheit vom Typ eines $C_{10-30}$-Alkylesters einer ungesättigten Carbonsäure einem Monomer der folgenden Formel (III) entspricht:

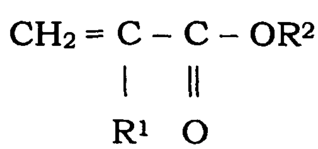

(III),

worin $R^1$ H oder $CH_3$ oder $C_2H_5$ (d.h. Acrylat-, Methacrylat- oder Ethacrylateinheiten) und vorzugsweise H (Acrylateinheiten) oder $CH_3$ (Methacrylateinheiten) und R eine $C_{10-30}$-Alkylgruppe und vorzugsweise eine $C_{12-22}$-Alkylgruppe bedeutet.

**18.** Emulsion nach Anspruch 7, wobei die vernetzte Homopolymere oder Copolymere mindestens eines kationischen Monomers mit ethylenischer Doppelbindung vom Ester- oder Amidtyp unter den folgenden Polymeren ausgewählt sind:

(1) Homopolymeren von Ammoniumacrylat und Copolymeren von Ammoniumacrylat und Acrylamid;
(2) Homopolymeren von mit Methylchlorid quaternisiertem Dimethylaminoethylmethacrylat oder Copolymeren von mit Methylchlorid quaternisiertem Dimethylaminoethylmethacrylat.

**19.** Emulsion nach einem der Ansprüche 1 bis 18, **dadurch gekennzeichnet, dass** das oder die vernetzte(n) Polymer (e) im Allgemeinen in einer Konzentration von 0,01 bis 10 Gew.-% und vorzugsweise 0,1 bis 5 Gew.-%, bezogen auf das Gesamtgewicht der Zusammensetzung, enthalten sind.

**20.** Emulsion nach einem der Ansprüche 1 bis 19, wobei das oder die unlöslichen, in mikronisierter Form vorliegenden organischen UV-Filter unter den organischen UV-Filtern vom Oxanilidtyp, Triazintyp, Triazoltyp, Vinylamidtyp oder Cinnamidtyp ausgewählt sind.

**21.** Emulsion nach Anspruch 20, wobei die UV-Filter vom Oxanilidtyp der folgenden Struktur entsprechen:

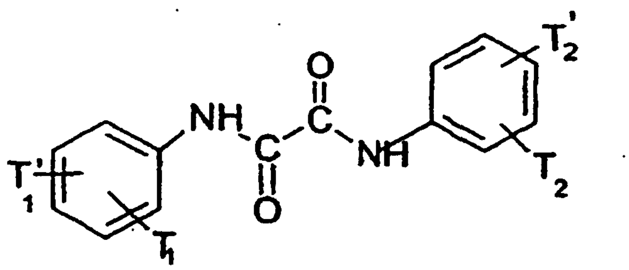

(1)

worin $T_1$, $T'_1$, $T_2$ und $T'_2$, die identisch oder voneinander verschieden sind, eine $C_{1-8}$-Alkylgruppe oder eine $C_{1-8}$-Alkoxygruppe bedeuten.

**22.** Emulsion nach Anspruch 20, wobei die UV-Filter vom Triazintyp unter den Verbindungen der folgenden allgemeinen Formel ausgewählt sind:

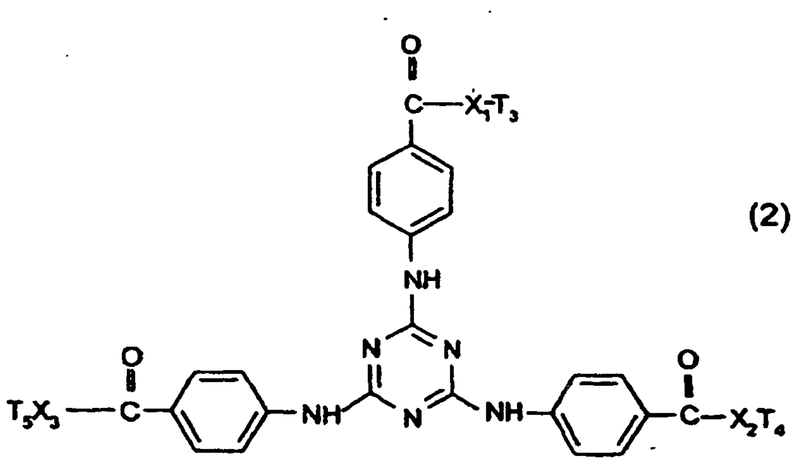

(2)

worin:

- die Gruppen $X_1$, $X_2$ und $X_3$, die identisch oder voneinander verschieden sind, Sauerstoff oder eine Gruppe -NZ bedeuten;
- die Gruppen Z, die identisch oder voneinander verschieden sind, Wasserstoff, eine geradkettige oder verzweigte $C_{1-18}$-Alkylgruppe oder eine $C_{5-12}$-Cycloalkylgruppe, die mit einer oder mehreren $C_{1-4}$-Alkylgruppen substituiert sein kann, bedeuten;
- die Gruppen $T_3$, $T_4$ und $T_5$, die identisch oder voneinander verschieden sind, ausgewählt sind unter: Wasserstoff; einem Alkalimetall; einer Ammoniumgruppe, die gegebenenfalls mit einer oder mehreren Alkyl- oder Hydroxyalkylgruppen substituiert ist; einer geradkettigen oder verzweigten $C_{1-18}$-Alkylgruppe; einer $C_{5-12}$-Cycloalkylgruppe, die gegebenenfalls mit einer oder mehreren $C_{1-4}$-Alkylgruppen substituiert ist; einer polyethoxylierten Gruppe mit 1 bis 6 Ethylenoxideinheiten, deren OH-Endgruppe methyliert ist; einer Gruppe der folgenden Formeln 3, 4 oder 5:

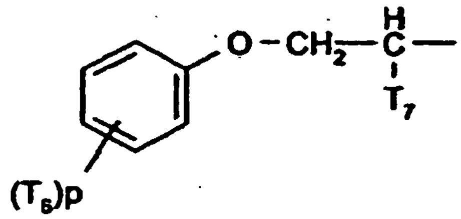

(3)

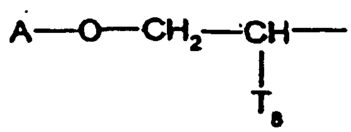

(4)

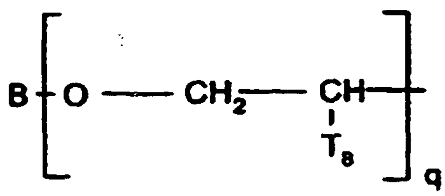

(5)

worin bedeuten:

- $T_6$ Wasserstoff oder Methyl;
- $T_7$ $C_{1-9}$-Alkyl;
- p 0 oder eine ganze Zahl von 1 bis 3;
- q eine ganze Zahl von 1 bis 10;
- A eine $C_{4-8}$-Alkylgruppe oder eine $C_{5-8}$-Cycloalkylgruppe;
- B ist ausgewählt unter: einer geradkettigen oder verzweigten $C_{1-8}$-Alkylgruppe; einer $C_{5-8}$-Cycloalkylgruppe; einer gegebenenfalls mit einer oder mehreren $C_{1-4}$-Alkylgruppen substituierten Arylgruppe;
- $T_8$ Wasserstoff oder Methyl.

**23.** Emulsion nach Anspruch 22, wobei das UV-Filter vom Triazintyp der folgenden Formel entspricht:

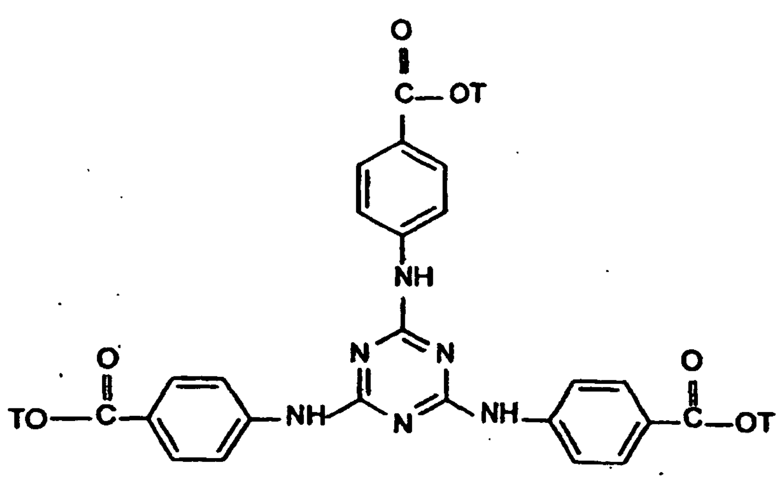

worin T eine 2-Ethylhexylgruppe bedeutet.

**24.** Emulsion nach Anspruch 22, wobei das UV-Filter vom Triazintyp der folgenden Formel entspricht:

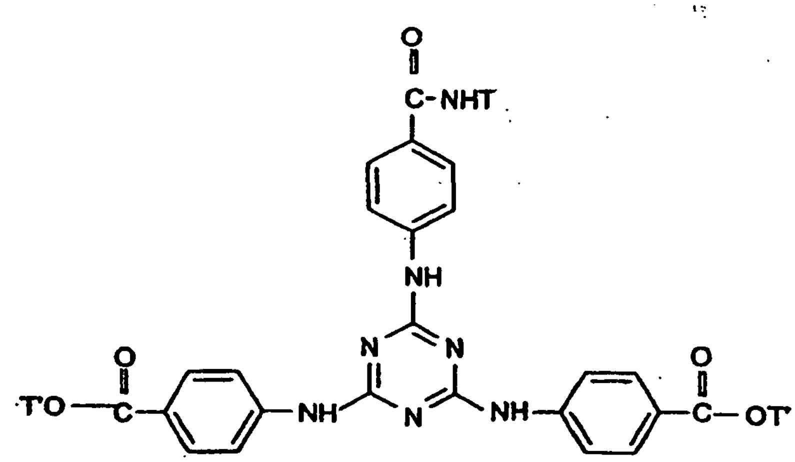

worin T' eine 2-Ethylhexylgruppe und T eine *t*-Butylgruppe bedeutet.

**25.** Emulsion nach Anspruch 20, wobei die UV-Filter vom Triazintyp unter den unlöslichen s-Triazinderivaten ausgewählt sind, die Benzalmalonat- und/oder Phenylcyanoacrylatgruppen enthalten.

**26.** Emulsion nach Anspruch 25, wobei die UV-Filter vom Triazintyp unter den folgenden Verbindungen ausgewählt sind:

- 2,4,6-Tris(diethyl-4'-aminobenzalmalonat)-s-triazin,
- 2,4,6-Tris(diisopropyl-4'-aminobenzalmalonat)-s-triazin,
- 2,4,6-Tris(dimethyl-4'-aminobenzalmalonat)-s-triazin,
- 2,4,6-Tris(ethyl-α-cyano-4-aminocinnamat)-s-triazin.

**27.** Emulsion nach Anspruch 20, wobei die UV-Filter vom Triazintyp unter den Verbindungen der folgenden Formel ausgewählt sind:

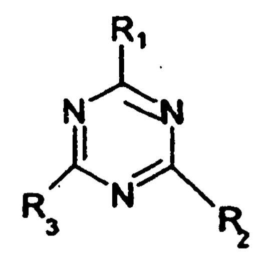

(6)

worin die Gruppen $R_1$, $R_2$ und $R_3$ unabhängig voneinander Phenyl, Phenoxy oder Pyrrolo bedeuten, wobei die Phenyl-, Phenoxy- oder Pyrrologruppen gegebenenfalls mit einem, zwei oder drei der folgenden Substituenten substituiert sind: OH, $C_{1-18}$-Alkyl oder $C_{1-18}$-Alkoxy, $C_{1-18}$-Carboxyalkyl, $C_{5-8}$-Cycloalkyl, Methylidencampher, $-(CH=CH)_n(CO)-OR^4$, wobei $R^4$ entweder $C_{1-18}$-Alkyl oder Cinnamyl bedeutet und n 0 oder 1 ist.

**28.** Emulsion nach Anspruch 20, wobei die UV-Filter vom Triazintyp unter den unlöslichen s-Triazinderivaten ausgewählt sind, die Benzotriazol- und/oder Benzothiazolgruppen enthalten.

**29.** Emulsion nach Anspruch 28, wobei die unlöslichen UV-Filter vom Triazintyp ausgewählt sind unter:

- 2,4,6-Tris[(3'-benzotriazol-2-yl-2'-hydroxy-5'-methyl)-phenylamino]-s-triazin,
- 2,4,6-Tris[(3'-benzotriazol-2-yl-2'-hydroxy-5'-*t*-octyl)-phenylamino]-s-triazin.

**30.** Emulsion nach Anspruch 20, wobei die unlöslichen organischen UV-Filter vom Triazoltyp der folgenden Formel 7 entsprechen:

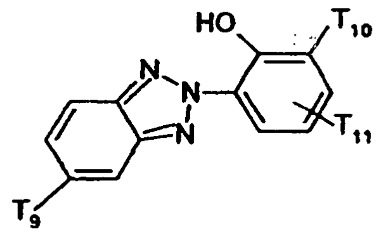

(7)

wobei $T_9$ ein Wasserstoffatom oder eine $C_{1-18}$-Alkylgruppe bedeutet und $T_{10}$ und $T_{11}$, die identisch oder voneinander verschieden sind, eine gegebenenfalls mit einer Phenylgruppe substituierte $C_{1-18}$-Alkylgruppe bedeuten.

**31.** Emulsion nach Anspruch 30, wobei die Verbindung der Formel 7 unter den folgenden Verbindungen ausgewählt ist:

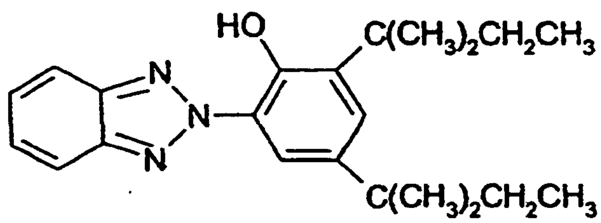

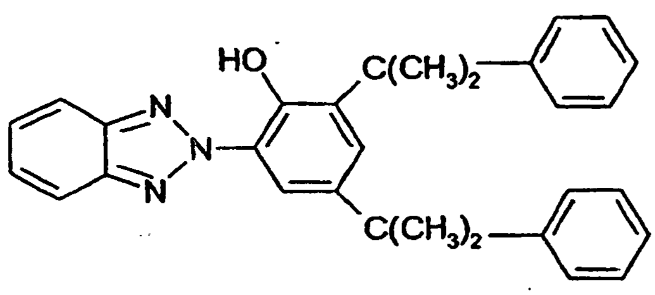

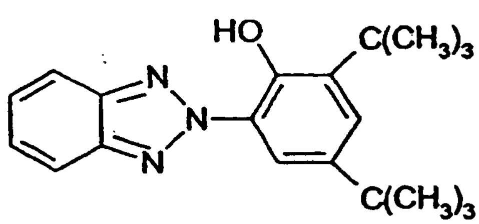

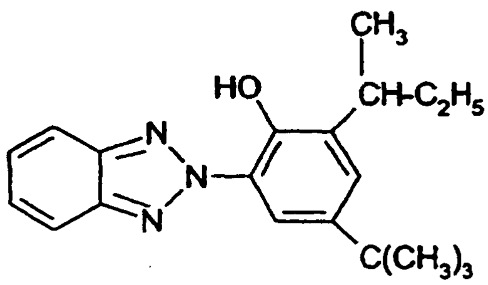

**32.** Emulsion nach Anspruch 20, wobei das unlösliche UV-Filter das [2,4'-Dihydroxy-3-(2H-benzotriazol-2-yl)-5-(1,1,3,3-tetramethylbutyl)-2'-n-octoxy-5'-benzoyl]diphenylmethan der folgenden Struktur ist:

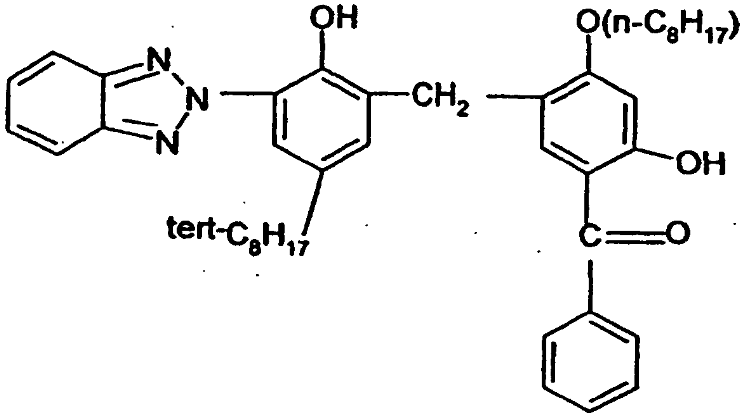

**33.** Emulsion nach Anspruch 20, wobei die organischen UV-Filter vom Triazoltyp unter den Methylen-bis(hydroxyphenylbenzotriazol)-Derivaten der folgenden Struktur ausgewählt sind:

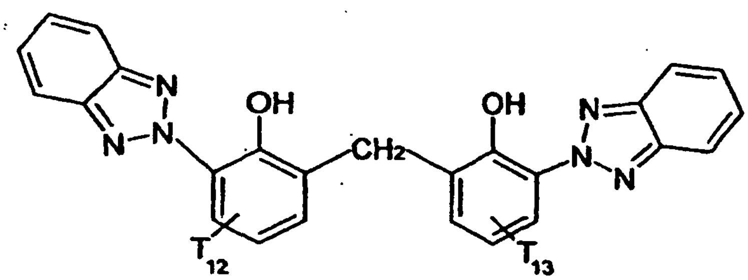

**(8)**

worin die Gruppen $T_{12}$ und $T_{13}$, die identisch oder voneinander verschieden sind, eine $C_{1-18}$-Alkylgruppe, die mit einer oder mehreren Gruppen substituiert sein kann, die unter den $C_{1-4}$-Alkylgruppen oder $C_{5-12}$-Cycloalkylgruppen ausgewählt sind, oder eine Arylgruppe bedeuten.

**34.** Emulsion nach Anspruch 33, wobei die Verbindung der Formel 8 unter den Verbindungen der folgenden Struktur ausgewählt ist:

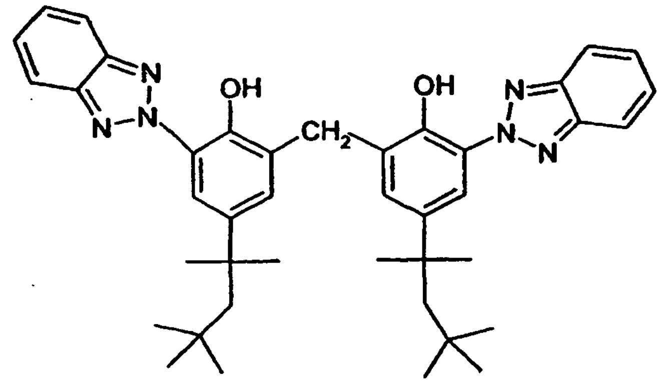

Verbindung (a)

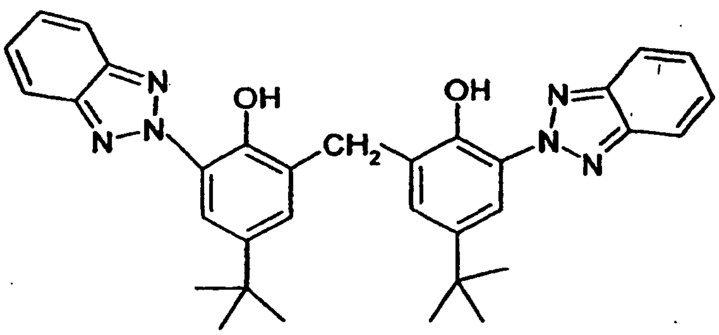

Verbindung (b)

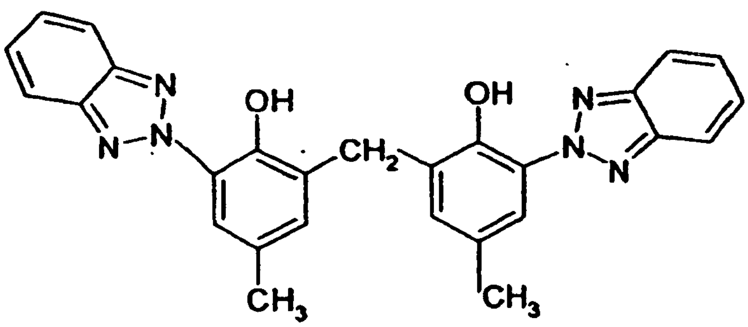

Verbindung (c)

**35.** Emulsion nach Anspruch 20, wobei die unlöslichen organischen Filter vom Vinylamidtyp der folgenden Formel entsprechen

$$T_{14}\text{-}(Y)_r\text{-}C(=O)\text{-}C(T_{15})=C(T_{16})\text{-}N(T_{17})(T_{18}) \quad (9),$$

worin $T_{14}$ eine $C_{1-18}$-Alkylgruppe und vorzugsweise eine $C_{1-5}$-Alkylgruppe, eine Phenylgruppe, die gegebenenfalls mit einer, zwei oder drei Gruppen substituiert ist, die ausgewählt sind unter: OH, $C_{1-18}$-Alkyl oder $C_{1-8}$-Alkoxy, oder $-C(=O)-OT_{19}$ bedeutet, wobei $T_{19}$ eine $C_{1-18}$-Alkylgruppe ist; die Gruppen $T_{15}$, $T_{16}$, $T_{17}$ und $T_{18}$, die identisch oder voneinander verschieden sind, eine $C_{1-18}$-Alkylgruppe und vorzugsweise eine $C_{1-5}$-Alkylgruppe oder ein Wasserstoffatom bedeuten; Y N oder O ist und r 0 oder 1 bedeutet.

**36.** Emulsion nach Anspruch 35, wobei die Verbindungen der Formel 9 ausgewählt sind unter:

- 4-Octylamino-3-penten-2-on;
- Ethyl-3-octylamino-2-butenoat;
- 3-Octylamino-1-phenyl-2-buten-1-on;
- 3-Dodecylamino-1-phenyl-2-buten-1-on.

**37.** Emulsion nach Anspruch 20, wobei die organischen Filter vom Cinnamamidtyp der folgenden Formel entsprechen:

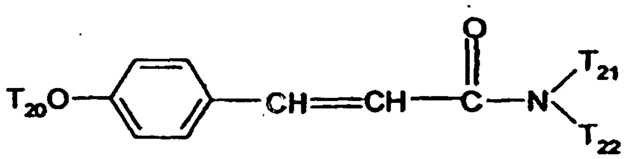

(10)

worin $OT_{20}$ eine Hydroxygruppe oder eine $C_{1-4}$-Alkoxygruppe ist, vorzugsweise Methoxy oder Ethoxy; $T_{21}$ Wasserstoff, $C_{1-4}$-Alkyl und vorzugsweise Methyl oder Ethyl bedeutet; $T_{22}$ eine Gruppe -$(CONH)_s$-phenyl, wobei s 0 oder 1 bedeutet und die Phenylgruppe mit einer, zwei oder drei Gruppen substituiert sein kann, die unter OH, $C_{1-18}$-Alkyl oder $C_{1-8}$-Alkoxy ausgewählt sind oder -C(=O)-$OT_{23}$ bedeutet, wobei $T_{23}$ eine $C_{1-18}$-Alkylgruppe und noch bevorzugter eine Phenylgruppe, 4-Methoxyphenyl oder Phenylaminocarbonyl ist.

**38.** Emulsion nach Anspruch 20, wobei das unlösliche UV-Filter ein Cinnamamid-Dimer ist.

**39.** Emulsion nach Anspruch 38, wobei das unlösliche UV-Filter die Verbindung der folgenden Struktur ist:

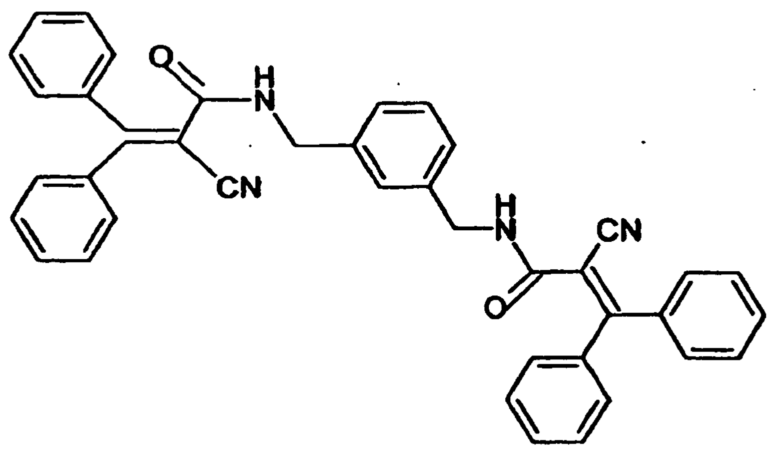

**40.** Emulsion nach einem der Ansprüche 1 bis 19, wobei die unlöslichen UV-Filter mehrwertige Metallsalze von organischen Sulfonfiltern oder Carboxyfiltern sind.

**41.** Emulsion nach Anspruch 40, wobei die unlöslichen UV-Filter unter den mehrwertigen Metallsalzen von sulfonierten Benzylidencampherderivaten; mehrwertigen Metallsalzen von sulfonierten Benzimidazolderivaten; und mehrwertigen Metallsalzen von Zimtsäurederivaten ausgewählt sind.

**42.** Emulsion nach einem der Ansprüche 1 bis 19,, wobei es sich bei den unlöslichen UV-Filtern um Komplexe von mehrwertigen Metallen oder Ammonium oder substituiertem Ammonium und organischen UV-A- und/oder UV-B-Filtern handelt.

**43.** Emulsion nach einem der vorhergehenden Ansprüche, **dadurch gekennzeichnet, dass** sie ferner ein oder mehrere zusätzliche, im UV-A- und/ oder UV-B-Bereich wirksame organische Filter enthält, die in mindestens einer der Phasen der Zusammensetzung löslich sind.

**44.** Emulsion nach Anspruch 43, **dadurch gekennzeichnet, dass** die zusätzlichen organischen Filter ausgewählt sind

unter: Zimtsäurederivaten, Salicylsäurederivaten, Campherderivaten, Triazinderivaten, Dibenzoylmethanderivaten, Benzophenonderivaten, β,β'-Diphenylacrylatderivaten, Benzimidazolderivaten, von α-Alkylstyrol abgeleiteten Dimeren, Bisbenzoazolylderivaten, p-Aminobenzoesäurederivaten, polymeren Filtern und Siliconfiltern.

**45.** Emulsion nach einem der vorhergehenden Ansprüche, **dadurch gekennzeichnet, dass** sie außerdem Pigmente oder Nanopigmente von Metalloxiden enthält, die gegebenenfalls umhüllt sind.

**46.** Emulsion nach Anspruch 45, **dadurch gekennzeichnet, dass** die Pigmente oder Nanopigmente unter den Oxiden von Titan, Zink, Eisen, Zirconium, Cer und deren Gemischen ausgewählt sind, wobei die Pigmente oder Nanopigmente gegebenenfalls umhüllt sind.

**47.** Emulsion nach einem der vorhergehenden Ansprüche, **dadurch gekennzeichnet, dass** sie außerdem mindestens ein Mittel zur Bräunung und/oder künstlichen Braunfärbung der Haut enthält.

**48.** Emulsion nach einem der vorhergehenden Ansprüche, **dadurch gekennzeichnet, dass** sie ferner mindestens einen Zusatzstoff enthält, der unter den Fettsubstanzen, organischen Lösungsmitteln, Verdickungsmitteln, beruhigenden Stoffen, Trübungsmitteln, Stabilisierungsmitteln, Emollientien, Antischaummitteln, Hydratisierungsmitteln, Parfums, Konservierungsmitteln, Polymeren, Füllstoffen, Maskierungsmitteln, Treibmitteln, Alkalisierungsmitteln oder Ansäuerungsmitteln ausgewählt ist.

**49.** Emulsion nach einem der Ansprüche 1 bis 48, **dadurch gekennzeichnet, dass** es sich um eine Zusammensetzung zum Schutz der menschlichen Epidermis oder ein Sonnenschutzmittel handelt und **dadurch**, dass sie als nichtionische Vesikeldispersion, Emulsion und insbesondere Emulsion vom Öl-in-Wasser-Typ, Creme, Milch, Gel, Gelcreme, Suspension, Dispersion, Pulver, fester Stift, Schaum oder Spray vorliegt.

**50.** Emulsion nach einem der Ansprüche 1 bis 48, **dadurch gekennzeichnet, dass** es sich um eine Zusammensetzung zum Schminken der Wimpern, Augenbrauen oder der Haut handelt und **dadurch**, dass sie in fester oder pastöser, wasserfreier oder wässriger Form, als Emulsion, Suspension oder Dispersion vorliegt.

**51.** Emulsion nach einem der Ansprüche 1 bis 48, **dadurch gekennzeichnet, dass** es sich um eine Zusammensetzung handelt, die zum Schutz der Haare gegen UV-Strahlung vorgesehen ist, und **dadurch**, dass sie als Haarwaschmittel, Lotion, Gel, Emulsion oder nichtionische Vesikeldispersion vorliegt.

**52.** Verwendung einer Emulsion nach einem der Ansprüche 1 bis 42 zur Herstellung von kosmetischen Zusammensetzungen zum Schutz der Haut und/oder der Haare gegen UV-Strahlung und insbesondere gegen Sonnenlicht.

**53.** Verwendung einer Emulsion ohne Emulgator nach einem der Ansprüche 1 bis 42 als Träger für die Herstellung einer kosmetischen oder dermatologischen Emulsion zum Lichtschutz, die mindestens ein in der Emulsion unlösliches, in einem der Ansprüche 2 bis 42 definiertes UV-Filter enthält, um im Hinblick auf das Filtervermögen die Wasserbeständigkeit zu erhöhen (Remanenz gegenüber Wasser).

## Claims

**1.** Cosmetic or dermatological emulsion without emulsifier, **characterized in that** it comprises:

a) at least one aqueous phase and
b) at least one fatty phase;
c) at least one photoprotective system capable of screening out UV rays, containing at least one organic UV-screening agent insoluble in the said emulsion, in micronized form, with a mean particle size ranging from 0.01 to 2 μm.
d) at least one crosslinked polymer of at least one monomer with ethylenic unsaturation and
e) optionally one surfactant which makes it possible to improve the dispersion of the particles of insoluble screening agent when the latter is obtained by grinding an insoluble UV-screening agent in the form of particles of coarse size in the presence of the said surfactant.

**2.** Emulsion according to Claim 1, where the insoluble UV-screening agent(s) in micronized insoluble form have a mean particle size ranging from 0.02 to 1.5 μm.

**3.** Emulsion according to Claim 2, where the insoluble UV-screening agent(s) in micronized insoluble form have a mean particle size ranging from 0.03 to 1.0 μm.

**4.** Emulsion according to any one of Claims 1 to 3, **characterized in that** the insoluble UV-screening agent(s) in micronized form can be obtained by a method of grinding the insoluble organic screening agent in the form of particles of coarse size in the presence of a surfactant which makes it possible to improve the dispersion of particles of insoluble screening agent.

**5.** Emulsion according to Claims 1 to 4, where the surfactant is chosen from alkyl polyglucosides having the structure $C_nH_{2n+1}O(C_6H_{10}O_5)_xH$ in which n is an integer from 8 to 16 and x is the average degree of polymerization of the unit $(C_6H_{10}O_5)$ and varies from 1.4 to 1.6.

**6.** Emulsion according to any one of Claims 1 to 5, **characterized in that** the surfactant is used at a concentration ranging from 1 to 50% by weight relative to the insoluble organic UV-screening agent in its micronized form.

**7.** Emulsion according to Claims 1 to 6 where the crosslinked polymer of at least one monomer with ethylenic unsaturation is chosen from the group consisting of:

(i) the crosslinked homopolymers or copolymers of at least one monomer with ethylenic unsaturation with a sulphonic function, in free or partially or completely salified form;
(ii) the crosslinked copolymers of at least one monomer with ethylenic unsaturation with a carboxylic acid function, in free or partially or completely salified form; or of an ester or amide derivative,
(iii) the crosslinked homopolymers or copolymers of at least one cationic monomer with ethylenic unsaturation of the ester or amide type.

**8.** Emulsion according to Claim 7, where the crosslinked homopolymers or copolymers of at least one monomer with ethylenic unsaturation with a sulphonic function are chosen from the group consisting of:

- the crosslinked homopolymers of 2-acrylamido-2-methylpropanesulphonic acid which are at least 90% neutralized;
- the crosslinked copolymers of 2-acrylamido-2-methylpropanesulphonic acid and of acrylamide which are partially or completely neutralized.

**9.** Emulsion according to Claim 7, where the crosslinked copolymers of at least one monomer with ethylenic unsaturation with a carboxylic acid function or of an ester or amide derivative are chosen from the non-ionic or anionic amphiphilic polymers comprising at least one fatty chain and at least one hydrophilic unit.

**10.** Emulsion according to Claim 9, where the non-ionic amphiphilic polymers are chosen from:

(a) the copolymers of $C_1$-$C_6$ alkyl methacrylates or acrylates and of amphiphilic monomers comprising at least one fatty chain;
(b) the copolymers of hydrophilic methacrylates or acrylates and of hydrophobic monomers comprising at least one fatty chain.

**11.** Emulsion according to Claim 9, where the anionic amphiphilic polymers are chosen from those whose hydrophilic unit consists of an ethylenically unsaturated anionic monomer, more particularly of a vinylcarboxylic acid.

**12.** Emulsion according to Claim 11, where the anionic amphiphilic polymers are chosen from those whose hydrophilic unit consists of an acrylic acid, a methacrylic acid or mixtures thereof and whose hydrophobic unit containing a fatty chain corresponds to the monomer having the following formula (I):

$$CH_2 = C (R') CH_2 O B_n R \qquad (I)$$

in which R' denotes H or $CH_3$, B denotes the ethyleneoxy radical, n is zero or denotes an integer from 1 to 100, R denotes a hydrocarbon radical chosen from the alkyl, arylalkyl, aryl, alkylaryl or cycloalkyl radicals, comprising 8 to 30 carbon atoms, preferably 10 to 24, and still more particularly from 12 to 18 carbon atoms.

**13.** Emulsion according to Claim 12, where in formula (I): R' denotes H, n is equal to 10, and R denotes a stearyl ($C_{18}$) radical.

**14.** Emulsion according to Claim 12 or 13, where the anionic amphiphilic polymers are formed from 20 to 60% by weight of acrylic acid and/or methacrylic acid, from 5 to 60% by weight of lower alkyl (meth)acrylates, from 2 to 50% by weight of allyl ether containing a fatty chain of formula (I), and from 0 to 1% by weight of a crosslinking agent.

**15.** Emulsion according to Claim 12 or 13, where the anionic amphiphilic polymers are chosen from the crosslinked terpolymers of methacrylic acid, ethyl acrylate, polyethylene glycol (10 EO) stearyl alcohol ether (steareth 10).

**16.** Emulsion according to Claim 13, where the anionic amphiphilic polymers are chosen from those comprising at least one hydrophilic unit of the olefinic unsaturated carboxylic acid type, and at least one hydrophobic unit of the ($C_{10}$-$C_{30}$) alkyl ester of unsaturated carboxylic acid type.

**17.** Emulsion according to Claim 16, where the amphiphilic polymers are preferably chosen from those whose hydrophilic unit of the olefinic unsaturated carboxylic acid type corresponds to the monomer having the following formula (II):

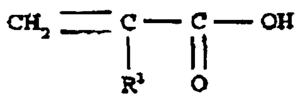

(II)

in which formula $R^1$ denotes H or $CH_3$ or $C_2H_5$, that is to say acrylic acid, methacrylic acid or ethacrylic acid units, and whose hydrophobic unit of the ($C_{10}$-$C_{30}$)alkyl ester of unsaturated carboxylic acid type corresponds to the monomer having the following formula (III):

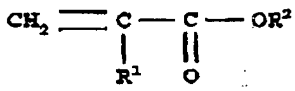

(III)

in which formula $R^1$ denotes H or $CH_3$ or $C_2H_5$ (that is to say acrylate, methacrylate or ethacrylate units) and preferably H (acrylate units) or $CH_3$ (methacrylate units), $R^2$ denoting a $C_{10}$-$C_{30}$, and preferably $C_{12}$-$C_{22}$, alkyl radical.

**18.** Emulsion according to Claim 7, where the crosslinked homopolymers or copolymers of at least one cationic monomer with ethylenic unsaturation of the ester or amide type are chosen from:

(1) the homopolymers of ammonium acrylate or the copolymers of ammonium acrylate and of acrylamide;
(2) the homopolymers of dimethylaminoethyl methacrylate quaternized with methyl chloride or the copolymers of dimethylaminoethyl methacrylate quaternized with methyl chloride.

**19.** Emulsion according to any one of Claims 1 to 18, in which the crosslinked polymer(s) are generally present at a concentration ranging from 0.01 to 10% by weight, and preferably from 0.1 to 5% by weight, relative to the total weight of the composition.

**20.** Emulsion according to any one of Claims 1 to 19, where the insoluble organic UV-screening agent(s) in micronized form are chosen from the insoluble organic UV-screening agents of the oxanilide type, of the triazine type, of the triazole type, of the vinylamide type and of the cinnamide type.

**21.** Emulsion according to Claim 20, where the UV-screening agents of the oxanilide type correspond to the structure:

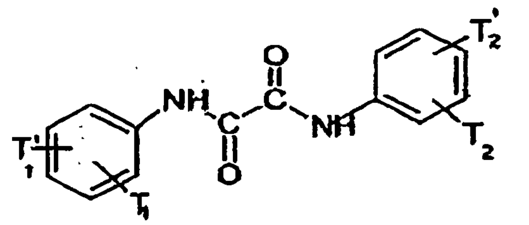

(1)

in which $T_1$, $T'_1$, $T_2$ and $T'_2$, which are identical or different, denote a $C_1$-$C_8$ alkyl radical or a $C_1$-$C_8$ alkoxy radical.

**22.** Emulsion according to Claim 20, where the UV-screening agents of the triazine type are chosen from those corresponding to the following general formula:

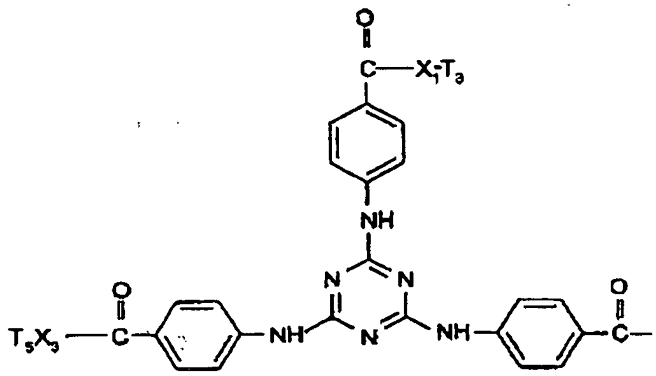

(2)

in which:

- $X_1$, $X_2$ and $X_3$, which are identical or different, represent oxygen or a radical -NZ-;
- the radicals Z, which are identical or different, denote hydrogen or a linear or branched $C_1$-$C_{18}$ alkyl radical, a $C_5$-$C_{12}$ cycloalkyl radical which may be substituted with one or more $C_1$-$C_4$ alkyl radicals;
- $T_3$, $T_4$ and $T_5$, which are identical or different, are chosen from hydrogen; an alkali metal; an ammonium radical which is optionally substituted with one or more alkyl or hydroxyalkyl radicals; a linear or branched $C_1$-$C_{18}$ alkyl radical; a $C_5$-$C_{12}$ cycloalkyl radical which is optionally substituted with one or more $C_1$-$C_4$ alkyl radicals; a polyoxyethylenated radical comprising from 1 to 6 ethylene oxide units and whose terminal OH group is methylated; a radical of the following formula (3), (4) or (5):

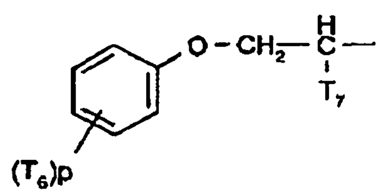

(3)

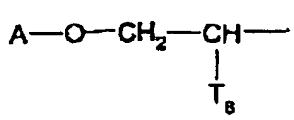

(4)

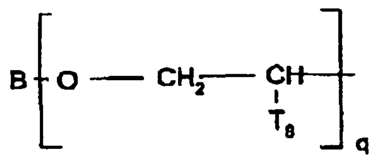

(5)

in which:

- $T_6$ is hydrogen or a methyl radical;
- $T_7$ is a $C_1$-$C_9$ alkyl radical;
- p is an integer ranging from 0 to 3;
- q is an integer ranging from 1 to 10;
- A is a $C_4$-$C_8$ alkyl radical or a $C_5$-$C_8$ cycloalkyl radical;
- B is chosen from a linear or branched $C_1$-$C_8$ alkyl radical; a $C_5$-$C_8$ cycloalkyl radical; an aryl radical which is optionally substituted with one or more $C_1$-$C_4$ alkyl radicals;
- $T_8$ is hydrogen or a methyl radical.

**23.** Emulsion according to Claim 22, where the UV-screening agent of the triazine type corresponds to the following formula:

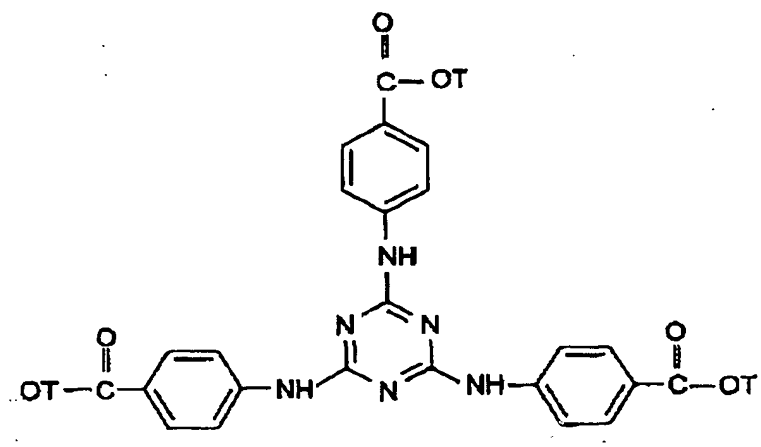

in which T denotes a 2-ethylhexyl radical.

**24.** Emulsion according to Claim 22, where the UV-screening agent of the triazine type corresponds to the following formula:

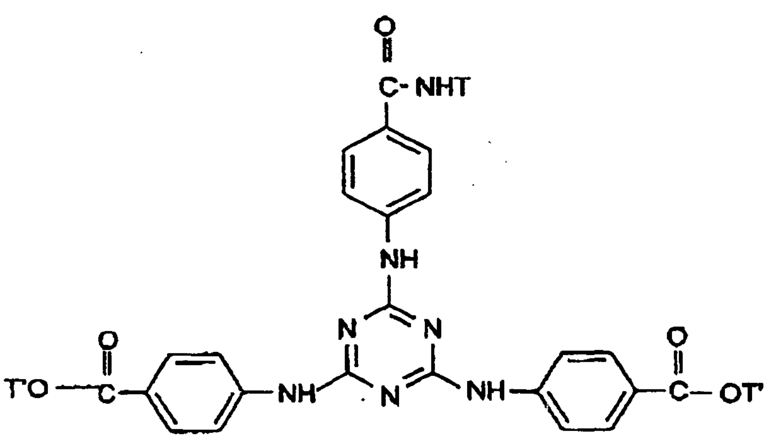

in which T' denotes a 2-ethylhexyl radical and T denotes a tert-butyl radical.

**25.** Emulsion according to Claim 20, where the UV-screening agents of the triazine type are chosen from the insoluble derivatives of s-triazine carrying benzalmalonate and/or phenylcyanoacrylate groups.

**26.** Emulsion according to Claim 25, where the UV-screening agents of the triazine type are chosen from the following compounds:

- 2,4,6-tris(diethyl 4'-aminobenzalmalonate)-s-triazine,
- 2,4,6-tris(diisopropyl 4'-aminobenzalmalonate)-s-triazine,
- 2,4,6-tris(dimethyl 4'-aminobenzalmalonate)-s-triazine,
- 2,4,6-tris(ethyl $\alpha$-cyano-4-aminocinnamate)-s-triazine.

**27.** Emulsion according to Claim 20, where the UV-screening agents of the triazine type are chosen from those corresponding to the following formula:

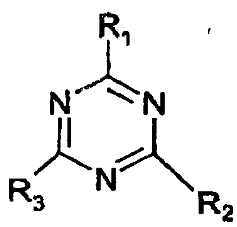

(6)

in which $R_1$, $R_2$, $R_3$ are independently phenyl, phenoxy, pyrrolo, in which the phenyl, phenoxy and pyrrolo are optionally substituted with one, two or three substituents chosen from OH, $C_1$-$C_{18}$ alkyl or alkoxy, $C_1$-$C_{18}$ carboxyalkyl, $C_5$-$C_8$ cycloalkyl, a methylidenecamphor group, a group $-(CH=CH)_n(CO)-OR_4$, with $R_4$ either $C_1$-$C_{18}$ alkyl or cinnamyl, and n is equal to 0 or 1.

**28.** Emulsion according to Claim 20, where the UV-screening agents of the triazine type are chosen from the insoluble derivatives of s-triazine carrying benzotriazole and/or benzothiazole groups.

**29.** Emulsion according to Claim 28, where the UV-screening agents of the triazine type are chosen from:

- 2,4,6-tris[(3'-benzotriazol-2-yl-2'-hydroxy-5'-methyl)phenylamino]-s-triazine,
- 2,4,6-tris[(3'-benzotriazol-2-yl-2'-hydroxy-5'-teroctyl)phenylamino]-s-triazine.

**30.** Emulsion according to Claim 20, where the organic UV-screening agents of the triazole type correspond to the following formula (7):

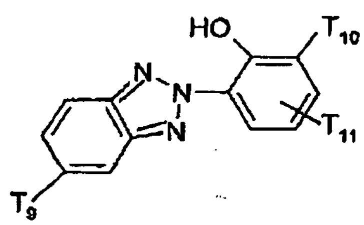

(7)

in which $T_9$ denotes a hydrogen atom or a $C_1$-$C_{18}$ alkyl radical; $T_{10}$ and $T_{11}$, which are identical or different, denote a $C_1$-$C_{18}$ alkyl radical which is optionally substituted with a phenyl.

**31.** Emulsion according to Claim 30, where the compound of formula (7) is chosen from the following compounds:

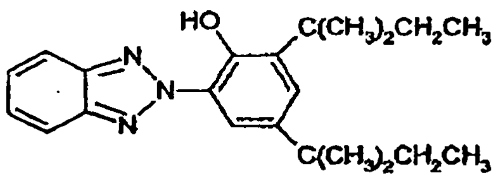

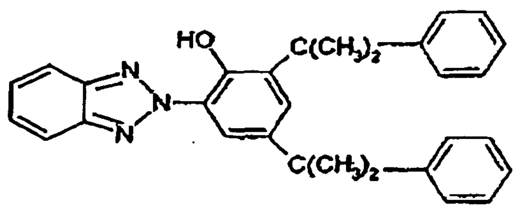

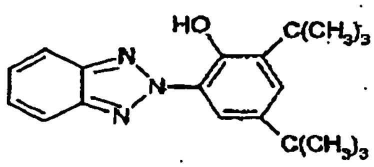

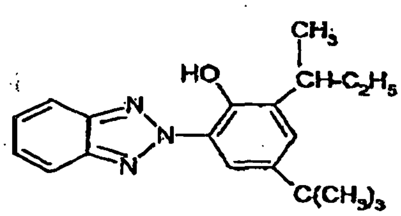

**32.** Emulsion according to Claim 20, where the insoluble UV-screening agent is [2,4'-dihydroxy-3-(2H-benzotriazol-2-yl)-5-(1,1,3,3-tetramethylbutyl)-2'-n-octoxy-5'-benzoyl]diphenylmethane having the structure:

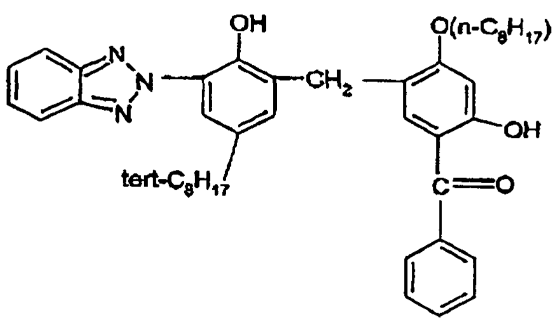

**33.** Emulsion according to Claim 20, where the organic UV-screening agents of the triazole type are chosen from the methylenebis(hydroxyphenylbenzo-triazole) derivatives having the following structure:

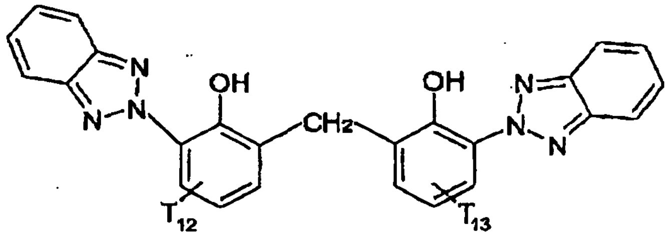

(8)

in which the radicals $T_{12}$ and $T_{13}$, which are identical or different, denote a $C_1$-$C_{18}$ alkyl radical which may be substituted with one or more radicals chosen from a $C_1$-$C_4$ alkyl, a $C_5$-$C_{12}$ cycloalkyl or an aryl residue.

**34.** Emulsion according to Claim 33, where the compound of formula (8) is chosen from the group consisting of the compounds having the following structure:

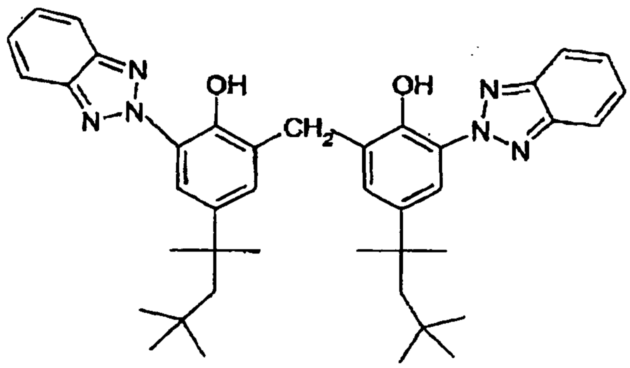

compound (a)

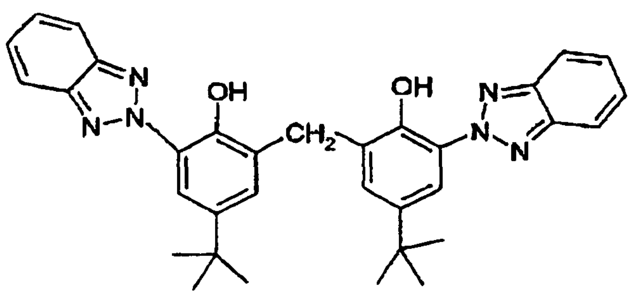

compound (b)

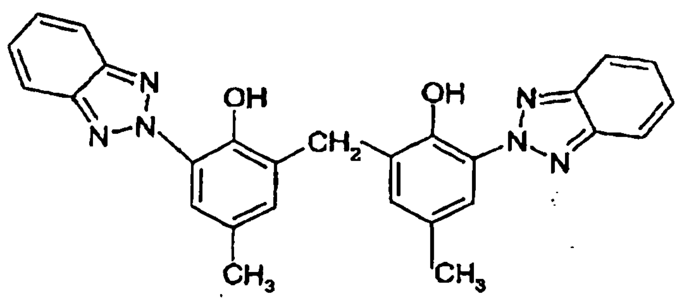

compound (c)

**35.** Emulsion according to Claim 20, where the organic screening agents of the vinylamide type correspond to the following formula:

$$T_{14}\text{-}(Y)r\text{-}C(=O)\text{-}C(T_{15})=C(T_{16})\text{-}N(T_{17})\ (T_{18}) \qquad (9)$$

in which $T_{14}$ is a $C_1$-$C_{18}$, preferably $C_1$-$C_5$, alkyl radical or a phenyl group which is optionally substituted with one, two or three radicals chosen from OH, $C_1$-$C_{18}$ alkyl, $C_1$-$C_8$ alkoxy, or a group -C(=O)-$OT_{19}$ where $T_{19}$ is a $C_1$-$C_{18}$ alkyl; $T_{15}$, $T_{16}$, $T_{17}$ and $T_{18}$, which are identical or different, denote a $C_1$-$C_{18}$, preferably $C_1$-$C_5$, alkyl radical or a hydrogen atom; Y is N or O and r is equal to 0 or 1.

**36.** Emulsion according to Claim 35 where the compounds of formula (9) are chosen from:

- 4-octylamino-3-penten-2-one;
- ethyl 3-octylamino-2-butenoate;
- 3-octylamino-1-phenyl-2-buten-1-one;
- 3-dodecylamino-1-phenyl-2-buten-1-one.

**37.** Emulsion according to Claim 20, where the organic screening agents of the cinnamamide type correspond to the following formula:

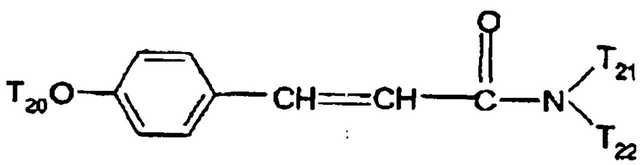

(10)

in which $OT_{20}$ is a hydroxyl or $C_1$-$C_4$ alkoxy, preferably methoxy or ethoxy, radical; $T_{21}$ is hydrogen, $C_1$-$C_4$ alkyl, preferably methyl or ethyl; $T_{22}$ is a group -(CONH)s-phenyl where s is equal to 0 or 1 and the phenyl group may be substituted with one, two or three groups chosen from OH, $C_1$-$C_{18}$ alkyl, $C_1$-$C_8$ alkoxy, or a group -C(=O)-$OT_{23}$ where $T_{23}$ is a $C_1$-$C_{18}$ alkyl and more preferably $T_{23}$ is a phenyl, 4-methoxyphenyl or phenylaminocarbonyl group.

**38.** Emulsion according to Claim 20, where the insoluble UV-screening agent is a cinnamamide dimer.

**39.** Emulsion according to Claim 38, where the insoluble UV-screening agent is the compound having the structure:

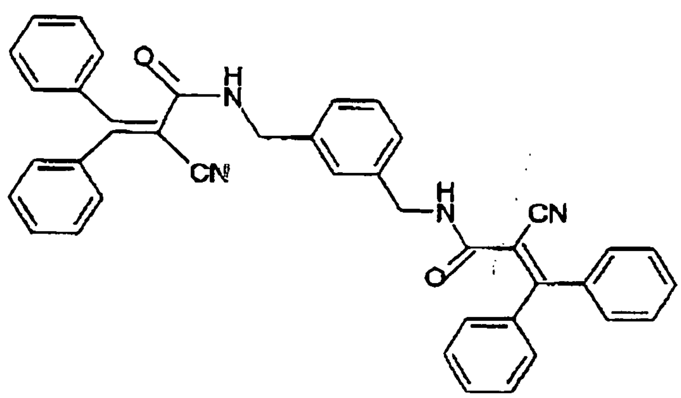

**40.** Emulsion according to any one of Claims 1 to 19, where the insoluble UV-screening agents are polyvalent metal salts of sulphonic or carboxylic organic UV-screening agents.

**41.** Emulsion according to Claim 40, where the insoluble UV-screening agents are polyvalent metal salts of sulphonated derivatives of benzylidenecamphor; the polyvalent metal salts of sulphonated derivatives of benzimidazole; the polyvalent metal salts of derivatives of cinnamic acid.

**42.** Emulsion according to any one of Claims 1 to 19, where the insoluble UV-screening agents are complexes of polyvalent metals or of ammonium or of ammonium substituted with organic UV-A and/or UV-B screening agents.

**43.** Emulsion according to any one of the preceding claims, **characterized in that** it comprises, in addition, one or more additional organic screening agents active in UV-A and/or UV-B, soluble in at least one of the phases of the emulsion.

**44.** Emulsion according to Claim 43, **characterized in that** the said additional organic screening agents are chosen from the cinnamic derivatives; the salicylic derivatives; the camphor derivatives; the triazine derivatives; the dibenzoyl-methane derivatives; the benzophenone derivatives; the β,β'-diphenyl acrylate derivatives; the benzimidazole derivatives; the dimers derived from α-alkylstyrene; the bisbenzoazolyl derivatives; the p-aminobenzoic acid derivatives; the polymer screening agents and silicone screening agents.

**45.** Emulsion according to any one of the preceding claims, **characterized in that** it comprises, in addition, pigments or nanopigments of metal oxides, coated or otherwise.

**46.** Emulsion according to Claim 45, **characterized in that** the said pigments or nanopigments are chosen from titanium oxides, zinc oxides, iron oxides, zirconium oxides or cerium oxides and mixtures thereof, coated or otherwise.

**47.** Emulsion according to any one of the preceding claims, **characterized in that** it comprises, in addition, at least one agent for tanning and/or for artificial tanning of the skin.

**48.** Emulsion according to any one of the preceding claims, **characterized in that** it comprises, in addition, at least one adjuvant chosen from fatty substances, organic solvents, thickeners, demulcents, opacifiers, stabilizers, emollients, antifoaming agents, moisturizing agents, perfumes, preservatives, polymers, sequestrants, propellants, alkalinizing or acidifying agents.

**49.** Emulsion according to any one of Claims 1 to 48, **characterized in that** it is a composition for protecting the human epidermis or an anti-sun composition and **in that** it is provided in the form of a nonionic vesicular dispersion, an emulsion, in particular an oil-in-water type emulsion, a cream, a milk, a gel, a gel cream, a suspension, a dispersion, a powder, a solid stick, a foam or a spray.

**50.** Emulsion according to any one of Claims 1 to 48, **characterized in that** it is a make-up composition for the eyelashes, the eyebrows or the skin and **in that** it is provided in solid or pasty, anhydrous or aqueous form, in the form of an emulsion, a suspension or a dispersion.

**51.** Emulsion according to any one of Claims 1 to 48, **characterized in that** it is a composition intended for protecting the hair against ultraviolet rays and **in that** it is provided in the form of a shampoo, a lotion, a gel, an emulsion, a nonionic vesicular dispersion.

**52.** Use of the emulsion defined in any one of Claims 1 to 42 for the manufacture of cosmetic compositions for protecting the skin and/or the hair against ultraviolet radiation, in particular solar radiation.

**53.** Use of an emulsion without emulsifier as defined in any one of Claims 1 to 42 as a carrier for the preparation of a photoprotective cosmetic or dermatological emulsion containing at least one insoluble UV-screening agent as defined in one of Claims 1 to 42, in order to improve the water resistance of its screening power (stability to water).